# EUROPEAN PATENT SPECIFICATION

(11) **EP 1 198 246 B1**
(45) Date of publication and mention of the grant of the patent: **28.04.2010**
(21) Application number: 01906979.8
(22) Date of filing: 02.02.2001
(51) Int. Cl.: A61K 39/09

(54) **METHODS FOR PREVENTING OR ATTENUATING PATHOANGIOGENIC CONDITIONS BY USING THE GBS-TOXIN (CM101) RECEPTOR AS A VACCINE**
VERFAHREN ZUR VORBEUGUNG UND LINDERUNG ANGIOGENER ERKRANKUNGEN DURCH IMPFUNG MIT DEM REZEPTOR FÜR GBS-TOXIN (CM101)
METHODES DE PREVENTION OU D'ATTENUATION D'ETATS PATHOLOGIQUES ANGIOGENIQUES

(30) Priority: 02.02.2000 US 179870 P
(43) Date of publication of application: 24.04.2002
(73) Proprietor: VANDERBILT UNIVERSITY, Nashville, TN 37240 (US)
(72) Inventor: HELLERQVIST, Carl G., Nashville, TN 37221 (US)
(74) Representative: Fleischer, Holm Herbert
(86) International application number: PCT/US2001/003662
(87) International publication number: WO 2001/056598

(56) References cited:
- WO-A-00/05375
- US-A- 5 010 062
- US-A- 5 858 991
- FU ET AL: "Expressional cloning of CM101 receptor gene from mammalian cells" PROCEEDINGS OF THE ANNUAL MEETING OF THE AMERICAN ASSOCIATION FOR CANCER RESEARCH, March 1999 (1999-03), XP002121519 cited in the application
- VORE DE R ET AL: "A PHASE I STUDY OF THE ANTI.NEOVASCULARIZATION DRUG CM-101" PROCEEDINGS OF THE ANNUAL MEETING OF THE AMERICAN SOCIETY OF CLINICAL ONCOLOGY,XX,XX, 1 March 1995 (1995-03-01), page 487 XP002071604
- HELLERQVIST C G ET AL: "ANTITUMOR EFFECTS OF GBS TOXIN: A POLYSACCHARIDE EXOTOXIN FROM GROUP B BETA-HEMOLYTIC STREPTOCOCCUS" JOURNAL OF CANCER RESEARCH AND CLINICAL ONCOLOGY,DE,SPRINGER INTERNATIONAL, BERLIN, vol. 120, no. 1/02, 1993, pages 63-70, XP000749401 ISSN: 0171-5216

### SEQUENCE LISTING

<110> Vanderbilt University
<120> Methods for Preventing or Attenuating Pathoangiogenic Conditions
<130> 22100-0100 WP 46126-252654
<150> US 60/179,870
   <151> 2000-02-02
<160> 4
<170> PatentIn version 3.0
<210> 1
   <211> 2930
   <212> DNA
   <213> Homo sapiens
<220>
   <221> CDS
   <222> (263)..(1870)
<400> 1
   gttcggtcga agccctcccc ttaattatgt gcaattcaag tccccactgc ccgcccgcaa      60
   gcccccactc atcctcgctg cgggcagggt ggcccctgca ctttacaagg gggtgcagga     120
   gcgggagacg gtcgtccgaa caccggctcc ccggcatgcg tagaccggcg ggcggagcgg     180
   gctcactttg cgccaatcct acgagaactc ccagaactcc gcttccctag tccaacccaa     240
   gccagagttg cccacaccta ag atg gcg gcg ggg gcg atg aca ccg ccc cgc     292
   ccg gtc cag cca gct cgg ccc ggg ggc ttc ggg ctg tcg ggc cgg cgc      340
   tcc ctt ctc tgc cag gtg gcg agt aca cct gct cac gta ggc gtc atg      388
   agg tct ccg gtt cga gac ctg gcc cgg aac gat ggc gag gag agc acg      436
   gac cgc acg cct ctt cta ccg ggc gcc cca cgg gcc gaa gcc gct cca      484
   gtg tgc tgc tct gct cgt tac aac tta gca att ttg gcc ttt ttt ggt      532
   ttc ttc att gtg tat gca tta cgt gtg aat ctg agt gtt gcg tta gtg      580
   gat atg gta gat tca aat aca act tta gaa gat aat aga act tcc aag      628
   gcg tgt cca gag cat tct gct ccc ata aaa gtt cat cat aat caa acg      676
   ggt aag aag tac caa tgg gat gca gaa act caa gga tgg att ctc ggt      724
   tcc ttt ttt tat ggc tac atc atc aca cag att cct gga gga tat gtt      772
   gcc agc aaa ata ggg ggg aaa atg ctg cta gga ttt ggg atc ctt ggc      820
   act gct gtc ctc acc ctg ttc act ccc att gct gca gat tta gga gtt      868
   gga cca ctc att gta ctc aga gca cta gaa gga cta gga gag ggt gtt      916
   aca ttt cca gcc atg cat gcc atg tgg tct tct tgg gct ccc cct ctt      964
   gaa aga agc aaa ctt ctt agc att tcg tat gca gga gca cag ctt ggg     1012
   aca gta att tct ctt cct ctt tct gga ata att tgc tac tat atg aat      1060
   tgg act tat gtc ttc tac ttt ttt ggt act att gga ata ttt tgg ttt      1108
   ctt ttg tgg atc tgg tta gtt agt gac aca cca caa aaa cac aag aga      1156
   att tcc cat tat gaa aag gaa tac att ctt tca tca tta aga aat cag      1204
   ctt tct tca cag aag tca gtg ccg tgg gta ccc att tta aaa tcc ctg      1252
   cca ctt tgg gct atc gta gtt gca cac ttt tct tac aac tgg act ttt      1300
   tat act tta ttg aca tta ttg cct act tat atg aag gag atc cta agg      1348
   ttc aat gtt caa gag aat ggg ttt tta tct tca ttg cct tat tta ggc      1396
   tct tgg tta tgt atg atc ctg tct ggt caa gct gct gac aat tta agg      1444
   gca aaa tgg aat ttt tca act tta tgt gtt cgc aga att ttt agc ctt      1492
   ata gga atg att gga cct gca gta ttc ctg gta gct gct ggc ttc att      1540
   ggc tgt gat tat tct ttg gcc gtt gct ttc cta act ata tca aca aca      1588
   ctg gga ggc ttt tgc tct tct gga ttt agc atc aac cat ctg gat att      1636
   gct cct tcg tat gct ggt atc ctc ctg ggc atc aca aat aca ttt gcc      1684
   act att cca gga atg gtt ggg ccc gtc att gct aaa agt ctg acc cct      1732
   gat aac act gtt gga gaa tgg caa acc gtg ttc tat att gct gct gct      1780
   att aat gtt ttt ggt gcc att ttc ttt aca cta ttc gcc aaa ggt gaa      1828
   gta caa aac tgg gct ctc aat gat cac cat gga cac aga cac              1870
   tgaaggaacc aataaataat cctgcctcta ttaatgtatt tttatttatc atgtaacctc   1930
   aaagtgcctt ctgtattgtg taagcattct atgtcttttt ttaattgtac ttgtattaga   1990
   tttttaaggc ctataatcat gaaatatcac tagttgccag aataataaaa tgaactgtgt   2050
   ttaattatga ataatatgta agctaggact tctactttag gttcacatac ctgcctgcta   2110
   gtcgggcaac atgaagtagg acagttctgt tgatttttta gggccatact aaagggaatg    2170
   agctgaaaca gacctcctga tacctttgct taattaaact agatgataat tctcaggtac   2230
   tgataaacac ctgttgttgt tcactttcct cataaaaatt gtcagctctc tctgacactt   2290
   agacctcaaa ctttagcatc tctgtggagc tgccatccac tgtataattt cgcctggcaa   2350
   ctggactgag gggagtgtgc ccaggcagct gccaagcact ccctccctgg cttcagggtc   2410
   agagtgccca gcgtttatca gaggcagcat ccaagcccag agccagtgtc gactcttcgg   2470
   ctggtgcctt tcctctgagg ggctatcaat gtgtagataa agccctgagt aggcaagagc   2530
   agtgagatcc actgctatgg tcttgataca tcctcaaact ttcccttccc agcacagagg   2590
   aatattggct ggcatgcaac ctgcaaaaga aaaatgcgaa gcggccgggc acggtggctc   2650
   atgcctgtaa tcccagcact ttggggggct gaggtgggcg aatcatgaga tcaggagttc   2710
   gagaccagcc tggccagcat ggtgaaaccc catctctact aaaaatacaa aaaattagct   2770
   gggcgtggtg acgggcgcct gtaatcccag atactcagga ggctgaggta ggagaatcac   2830
   ttgaacctgg gaggtggaag ttgcagtgaa ccaagatcac gccactgcac tccagcctgg   2890
   gcgatggagc gagactccaa ctcaaaaaaa aaaaaaaaaa                         2930
<210> 2
   <211> 536
   <212> PRT
   <213> Homo sapiens
<400> 2
<210> 3
   <211> 2844
   <212> DNA
   <213> Ovis sp.
<220>
   <221> CDS
   <222> (84)..(1568)
<400> 3
   cccgggggcg gggggcttcg gcggtcccgc tggagctctc ttttccgcgg agcaggtttg      60
   cgccgtagct ccctgaaggc atc atg aag tcc ccg gtt tcg gac tta gcc ccg     113
                             Met Lys Ser Pro Val Ser Asp Leu Ala Pro
                             1               5                   10
   agc gac ggc gag gag ggc tcg gac cgc aca ccg ctc ctg cag cgc gcc      161
   Ser Asp Gly Glu Glu Gly Ser Asp Arg Thr Pro Leu Leu Gln Arg Ala
                   15                  20                  25
   ccg cgg gcg gaa ccc gct cca gta tgc tgc tct gct cgt tac aac cta      209
   Pro Arg Ala Glu Pro Ala Pro Val Cys Cys Ser Ala Arg Tyr Asn Leu
               30                  35                  40
   gca ttt ttg tcc ttt ttt ggt ttc ttc gtt ctc tat tca tta cgg gtg      257
   Ala Phe Leu Ser Phe Phe Gly Phe Phe Val Leu Tyr Ser Leu Arg Val
           45                  50                  55
   aat ctg agc gtt gca cta gtg gac atg gtg gat tca aac aca act gcc      305
   Asn Leu Ser Val Ala Leu Val Asp Met Val Asp Ser Asn Thr Thr Ala
       60                  65                  70
   aaa gat aat aga acg tcc tac gag tgt gca gag cat tct gct ccc ata      353
   Lys Asp Asn Arg Thr Ser Tyr Glu Cys Ala Glu His Ser Ala Pro Ile
   75                  80                  85                  90
   aaa gtt ctt cac aac caa acg ggt aaa aag tac cgg tgg gat gca gaa      401
   Lys Val Leu His Asn Gln Thr Gly Lys Lys Tyr Arg Trp Asp Ala Glu
                   95                  100                 105
   act caa gga tgg att ctc gga tct ttt ttc tat ggc tac atc atc aca      449
   Thr Gln Gly Trp Ile Leu Gly Ser Phe Phe Tyr Gly Tyr Ile Ile Thr
               110                 115                 120
   caa att cct gga gga tat gtt gcc agc aga agt ggg ggg aag ctg ttg      497
   Gln Ile Pro Gly Gly Tyr Val Ala Ser Arg Ser Gly Gly Lys Leu Leu
           125                 130                 135
   cta gga ttc ggg atc ttt gct aca gct atc ttc acc ctg ttc act ccc      545
   Leu Gly Phe Gly Ile Phe Ala Thr Ala Ile Phe Thr Leu Phe Thr Pro
       140                 145                 150
   ctc gct gca gat ttc gga gtc gga gcc ctt gtt gca ctc agg gca cta      593
   Leu Ala Ala Asp Phe Gly Val Gly Ala Leu Val Ala Leu Arg Ala Leu
   155                 160                 165                 170
   gaa ggg cta gga gag ggt gtc aca tat cca gcc atg cat gcc atg tgg      641
   Glu Gly Leu Gly Glu Gly Val Thr Tyr Pro Ala Met His Ala Met Trp
                   175                 180                 185
   tct tca tgg gct ccc cct ctt gaa aga agc aag ctt ctg agt att tca      689
   Ser Ser Trp Ala Pro Pro Leu Glu Arg Ser Lys Leu Leu Ser Ile Ser
               190                 195                 200
   tat gca gga gca caa ctt ggg aca gta gtt tct ctt cct ctt tct gga      737
   Tyr Ala Gly Ala Gln Leu Gly Thr Val Val Ser Leu Pro Leu Ser Gly
           205                 210                 215
   gta att tgc tac tat atg aat tgg act tat gtc ttc tat ttc ttt ggc      785
   Val Ile Cys Tyr Tyr Met Asn Trp Thr Tyr Val Phe Tyr Phe Phe Gly
       220                 225                 230
   att gtt gga atc atc tgg ttt att tta tgg atc tgc tta gtt agt gat      833
   Ile Val Gly Ile Ile Trp Phe Ile Leu Trp Ile Cys Leu Val Ser Asp
   235                 240                 245                 250
   aca cca gaa act cac aag aca atc act ccc tat gaa aag gag tat att      881
   Thr Pro Glu Thr His Lys Thr Ile Thr Pro Tyr Glu Lys Glu Tyr Ile
                   255                 260                 265
   ctt tca tca tta aaa aat cag ctc tct tca cag aag tca gtg ccg tgg      929
   Leu Ser Ser Leu Lys Asn Gln Leu Ser Ser Gln Lys Ser Val Pro Trp
               270                 275                 280
   ata cct atg ctg aaa tca ctg cca ctt tgg gct att gtc gtt gca cat      977
   Ile Pro Met Leu Lys Ser Leu Pro Leu Trp Ala Ile Val Val Ala His
           285                 290                 295
   ttt tct tac aac tgg act ttt tat act ttg ttg acc tta ttg cct act     1025
   Phe Ser Tyr Asn Trp Thr Phe Tyr Thr Leu Leu Thr Leu Leu Pro Thr
       300                 305                 310
   tac atg aag gaa gtc cta agg ttc aat att caa gag aat ggg ttt tta     1073
   Tyr Met Lys Glu Val Leu Arg Phe Asn Ile Gln Glu Asn Gly Phe Leu
   315                 320                 325                 330
   tct gca gtc cct tat tta ggt tgt tgg tta tgt atg atc ctg tcg ggt     1121
   Ser Ala Val Pro Tyr Leu Gly Cys Trp Leu Cys Met Ile Leu Ser Gly
                   335                 340                 345
   caa gct gct gac aat tta agg gca aga tgg aat ttt tca act ctg tgg     1169
   Gln Ala Ala Asp Asn Leu Arg Ala Arg Trp Asn Phe Ser Thr Leu Trp
               350                 355                 360
   gtt cga aga gtt ttt agc ctt ata ggg atg att gga cct gcg ata ttc     1217
   Val Arg Arg Val Phe Ser Leu Ile Gly Met Ile Gly Pro Ala Ile Phe
           365                 370                 375
   ctg gtt gcc gca gga ttt ata ggc tgt gat tat tcc ttg gct gtt gca     1265
   Leu Val Ala Ala Gly Phe Ile Gly Cys Asp Tyr Ser Leu Ala Val Ala
       380                 385                 390
   ttc cta acc ata tca aca acc ctg gga ggc ttt tgc tct tct gga ttt     1313
   Phe Leu Thr Ile Ser Thr Thr Leu Gly Gly Phe Cys Ser Ser Gly Phe
   395                 400                 405                 410
   agc atc aac cat ctg gac att gct cct tcg tat gct ggt att ctc ctg     1361
   Ser Ile Asn His Leu Asp Ile Ala Pro Ser Tyr Ala Gly Ile Leu Leu
                   415                 420                 425
   ggc atc aca aat acc ttt gcc act att cct gga atg att ggg ccc atc     1409
   Gly Ile Thr Asn Thr Phe Ala Thr Ile Pro Gly Met Ile Gly Pro Ile
               430                 435                 440
   att gcc aga agt ctt acc cct gag aac act att gga gaa tgg caa act     1457
   Ile Ala Arg Ser Leu Thr Pro Glu Asn Thr Ile Gly Glu Trp Gln Thr
           445                 450                 455
   gtt ttc tgc atc gct gct gct atc aat gta ttt ggt gcc att ttc ttc     1505
   Val Phe Cys Ile Ala Ala Ala Ile Asn Val Phe Gly Ala Ile Phe Phe
       460                 465                 470
   aca cta ttc gcc aaa ggt gaa gtg caa aac tgg gcc atc agt gat cac     1553
   Thr Leu Phe Ala Lys Gly Glu Val Gln Asn Trp Ala Ile Ser Asp His
   475                 480                 485                 490
   caa gga cac aga aac tgaaggaacc aataaataat cctgtctcta ttaatgtatc     1608
   Gln Gly His Arg Asn
                   495
   tttgtttatc atgtaaccta aaagtgcctt tgatatttta atgtgtaagc aatctatata   1668
   caagataaaa ttgtactaga aaaattgtgt tagatttgta aggcttgtaa tcatgaaatg   1728
   tcactagttg ccatataagc aaaattagct atttttaatt attattaacc cgtttgctgg   1788
   aacttacaat tcagggtcac atatctggct gcaagtcagg caacccacaa taggggagtt   1848
   ctatttattt ataagaccat acctaaagag atgagctgaa atagaccctt ctataccttt   1908
   gcttaattaa ggtggataat aattctcagg tcttgttaaa catctgtttt tgtacacctt   1968
   cctcaaaaaa ttatttgtca tcagcaatcc ctgacatgta ggtctcaaac tttagcctct   2028
   ccacggagct ggcagccact gtatcattca gcctggcaac ttcactgagg gaagcatgcc   2088
   caggcagctg ccacatgtcc cctctctggc ttcagggaca gtgcccagca cttaggcagc   2148
   atccaagacc agggtcagcg ccaaggcttt ggacggtatt cttcccctgg ggctgttaat   2208
   gtgtggatga agccctgagc caacagggac agcgcgatcc acagtcatgg tttccatgca   2268
   ccctctccct tcccttccca gcacactgga gtattgcctg gcatgtaacc tgcaaaagaa   2328
   agtgtgatgc ctaattagcc acatataaca tcatccttga tgatcctacc ttcacatgga   2388
   tcagagtata aatcttcaag tcctgtgttc taggagctac accagaataa ttaaaatata   2448
   aaaagaaaca aaacattttt tctgtctgac acctaagtgt ctggttgcag ttcaaggtta   2508
   aagtgacttc tacttcacat aacctgcaac cggtggtgta atcatcttta gtgttggttt   2568
   cttaaatctt atttttccag tttttcctgg accatcttcc agtggttttg agcatgcttt   2628
   gagggcattt atgtgattta gaacttgatt aatgtttcac tgtgtatgtt caacactacc   2688
   tgtaatattt taactaaagc tatttaatgt aatatgatgt gtatacattc tgtaaattaa   2748
   tttttaaatc tgtaaatagc tttaagttgc tatggtgata tttcttttac aaatcaaaat   2808
   aaatcttttt ggaatgataa aaaaaaaaaa aaaaaa                             2844
<210> 4
   <211> 495
   <212> PRT
   <213> Ovis sp.
<400> 4
   Met Lys Ser Pro Val Ser Asp Leu Ala Pro Ser Asp Gly Glu Glu Gly
   1               5                   10                  15
   Ser Asp Arg Thr Pro Leu Leu Gln Arg Ala Pro Arg Ala Glu Pro Ala
               20                  25                  30
   Pro Val Cys Cys Ser Ala Arg Tyr Asn Leu Ala Phe Leu Ser Phe Phe
           35                  40                  45
   Gly Phe Phe Val Leu Tyr Ser Leu Arg Val Asn Leu Ser Val Ala Leu
       50                  55                  60
   Val Asp Met Val Asp Ser Asn Thr Thr Ala Lys Asp Asn Arg Thr Ser
   65                  70                  75                  80
   Tyr Glu Cys Ala Glu His Ser Ala Pro Ile Lys Val Leu His Asn Gln
                   85                  90                  95
   Thr Gly Lys Lys Tyr Arg Trp Asp Ala Glu Thr Gln Gly Trp Ile Leu
               100                 105                 110
   Gly Ser Phe Phe Tyr Gly Tyr Ile Ile Thr Gln Ile Pro Gly Gly Tyr
           115                 120                 125
   Val Ala Ser Arg Ser Gly Gly Lys Leu Leu Leu Gly Phe Gly Ile Phe
       130                 135                 140
   Ala Thr Ala Ile Phe Thr Leu Phe Thr Pro Leu Ala Ala Asp Phe Gly
   145                 150                 155                 160
   Val Gly Ala Leu Val Ala Leu Arg Ala Leu Glu Gly Leu Gly Glu Gly
                   165                 170                 175
   Val Thr Tyr Pro Ala Met His Ala Met Trp Ser Ser Trp Ala Pro Pro
               180                 185                 190
   Leu Glu Arg Ser Lys Leu Leu Ser Ile Ser Tyr Ala Gly Ala Gln Leu
           195                 200                 205
   Gly Thr Val Val Ser Leu Pro Leu Ser Gly Val Ile Cys Tyr Tyr Met
       210                 215                 220
   Asn Trp Thr Tyr Val Phe Tyr Phe Phe Gly Ile Val Gly Ile Ile Trp
   225                 230                 235                 240
   Phe Ile Leu Trp Ile Cys Leu Val Ser Asp Thr Pro Glu Thr His Lys
                   245                 250                 255
   Thr Ile Thr Pro Tyr Glu Lys Glu Tyr Ile Leu Ser Ser Leu Lys Asn
               260                 265                 270
   Gln Leu Ser Ser Gln Lys Ser Val Pro Trp Ile Pro Met Leu Lys Ser
           275                 280                 285
   Leu Pro Leu Trp Ala Ile Val Val Ala His Phe Ser Tyr Asn Trp Thr
       290                 295                 300
   Phe Tyr Thr Leu Leu Thr Leu Leu Pro Thr Tyr Met Lys Glu Val Leu
   305                 310                 315                 320
   Arg Phe Asn Ile Gln Glu Asn Gly Phe Leu Ser Ala Val Pro Tyr Leu
                   325                 330                 335
   Gly Cys Trp Leu Cys Met Ile Leu Ser Gly Gln Ala Ala Asp Asn Leu
               340                 345                 350
   Arg Ala Arg Trp Asn Phe Ser Thr Leu Trp Val Arg Arg Val Phe Ser
           355                 360                 365
   Leu Ile Gly Met Ile Gly Pro Ala Ile Phe Leu Val Ala Ala Gly Phe
       370                 375                 380
   Ile Gly Cys Asp Tyr Ser Leu Ala Val Ala Phe Leu Thr Ile Ser Thr
   385                 390                 395                 400
   Thr Leu Gly Gly Phe Cys Ser Ser Gly Phe Ser Ile Asn His Leu Asp
                   405                 410                 415
   Ile Ala Pro Ser Tyr Ala Gly Ile Leu Leu Gly Ile Thr Asn Thr Phe
               420                 425                 430
   Ala Thr Ile Pro Gly Met Ile Gly Pro Ile Ile Ala Arg Ser Leu Thr
           435                 440                 445
   Pro Glu Asn Thr Ile Gly Glu Trp Gln Thr Val Phe Cys Ile Ala Ala
       450                 455                 460
   Ala Ile Asn Val Phe Gly Ala Ile Phe Phe Thr Leu Phe Ala Lys Gly
   465                 470                 475                 480
   Glu Val Gln Asn Trp Ala Ile Ser Asp His Gln Gly His Arg Asn
                   485                 490                 495

## Description

### TECHNICAL FIELD

This invention relates to methods and compositions for preventing or ameliorating medical conditions arising from the formation of pathological neovasculature. These conditions include cancer, scarring during wound healing, keloid formation, chronic wounds, gliosis during repair of nerve injury, reperfusion injury, rheumatoid arthritis, psoriasis and atherosclerosis.

### BACKGROUND

Cancer is the second leading cause of death in the United States, second only to heart disease (which is frequently due in part to atherosclerosis). Since 1990 approximately 12 million new cases of cancer have been diagnosed and five million persons have died of cancer in the United States.

Neural injury can result in death or profound disability such as loss of movement, impaired sensory perception, loss of cognitive functions, seizures, and emotional and personality disorders.

Rheumatoid arthritis (RA) and psoriasis are prevalent chronic inflammatory diseases propagated by inflammatory angiogenesis. RA affects approximately 1-2% of the world's population. RA sufferers often experience pain and impaired mobility, and as a group they have twice the mortality rate of their unaffected counterparts. Approximately 1-3% of United States residents and an even higher percentage of Northern Europeans suffer from psoriasis, a disease in which the skin develops recurrent erythematous plaques that burn and itch.

Chronic wounds or skin ulcers are major problems in diabetic and geriatric populations. Poor healing of acute wounds inflicted by accidents or surgical incisions and the formation of wound-associated scars seriously impair recovery from such events. Reperfusion injuries cause damage to transplanted organs as well as tissues near the site of surgical intervention, stroke or heart attack.

Fu et al. report in an abstract (No. 3677) in "Proceedings of the annual meeting of the American Society for Cancer Research" (03-1999) about the expressional cloning of CM101 receptor gene from mammalian cells. Said receptor gene encodes a 495 amino acid protein of approx. 65 KDa having 7 transmembrane domains and 7 potential N-glycosylation sites. This receptor appears to be involved in tumor pathogenesis, whereby tumor growth can be inhibited by binding CM101, a polysaccharide to said receptor.

WO 00/05375 discloses novel group B β-hemolytic Streptococci (GBS) receptors and fragments thereof and their use for searching and targeting compounds binding to said receptors as well as methods and use of said receptors, fragments of binding compounds for preparation of medicaments.

There is a need for a prophylaxis that would prevent cancer, gliosis during repair of nerve injury, chronic inflammatory diseases such as rheumatoid arthritis and psoriasis, scarring during wound healing, keloid formation, chronic wounds, reperfusion injury and atherosclerosis. There is also a need for an effective and safe therapy for each of these medical conditions.

The present invention provides a composition and its medical uses as defined in the claims.

### SUMMARY OF THE INVENTION

The present disclosure provides a method for preventing or protecting against pathoangiogenic conditions by administering one or more Group B β-hemolytic *Streptococci* (GBS) toxin receptors or immunogenic fragments thereof to a mammal in an amount sufficient to induce or maintain an immune response to at least one of the GBS toxin receptors.

The present disclosure also provides a method for decreasing the incidence of, ameliorating, lessening the severity of, or attenuating pathoangiogenic conditions by administering one or more GBS toxin receptors or immunogenic fragments thereof to a mammal in an amount sufficient to induce or maintain an immune response to at least one of the GBS toxin receptors.

Disclosed is also a vaccine comprising one or more GBS toxin receptors or immunogenic fragments thereof in combination with a pharmaceutically acceptable excipient.

The pathoangiogenic conditions that can be prevented or attenuated by the methods and compositions of the present invention include cancer, scarring during wound healing, keloid formation, chronic wounds, gliosis during repair of nerve injury, reperfusion injury, rheumatoid arthritis, psoriasis and atherosclerosis.

Preferred GBS toxin receptors are HP59 and SP55 and substantially identical variants thereof. Mammals treated with the compositions of the present invention may be additionally treated with GBS toxin, with immunocompatible antibodies directed at the GBS toxin receptor, or with autologous activated GBS toxin receptor-recognizing T cells.

Yet another aspect of the present invention is a method for making a composition for the treatment and/or prevention of pathoangiogenic conditions. This method involves providing one or more GBS toxin receptors or immunogenic fragments thereof as defined in claim 1, and formulating it in a pharmaceutically acceptable excipient. An adjuvant may optionally be provided.

### BRIEF DESCRIPTION OF THE FIGURES

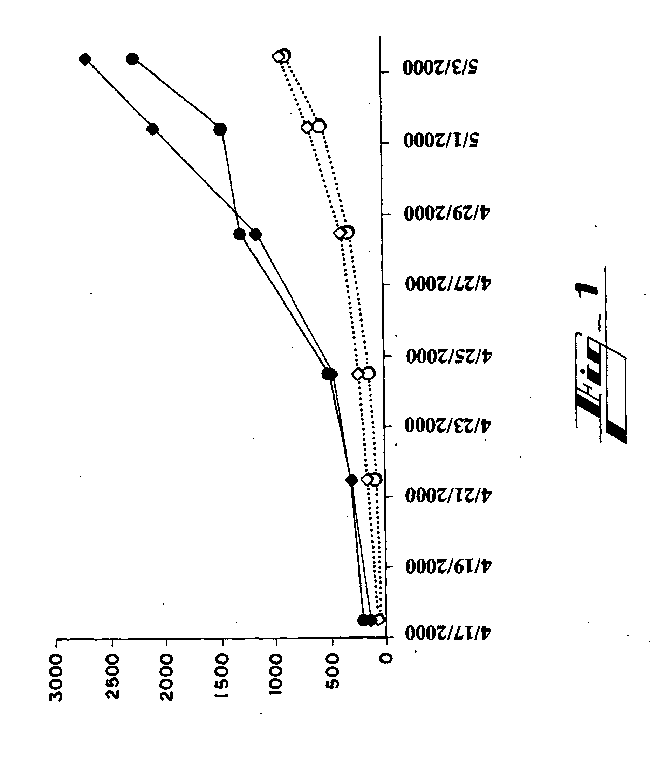

Fig. 1 presents growth curves for Lewis Lung tumors in control mammals and in male and female mammals immunized with a composition of the present invention. The y-axis represents tumor volume and the x-axis represents the day the tumor volume was measured. Male controls are denoted by blackened diamonds, female controls are denoted by blackened circles; male immunized are denoted by clear circles and female immunized are denoted by clear diamonds.

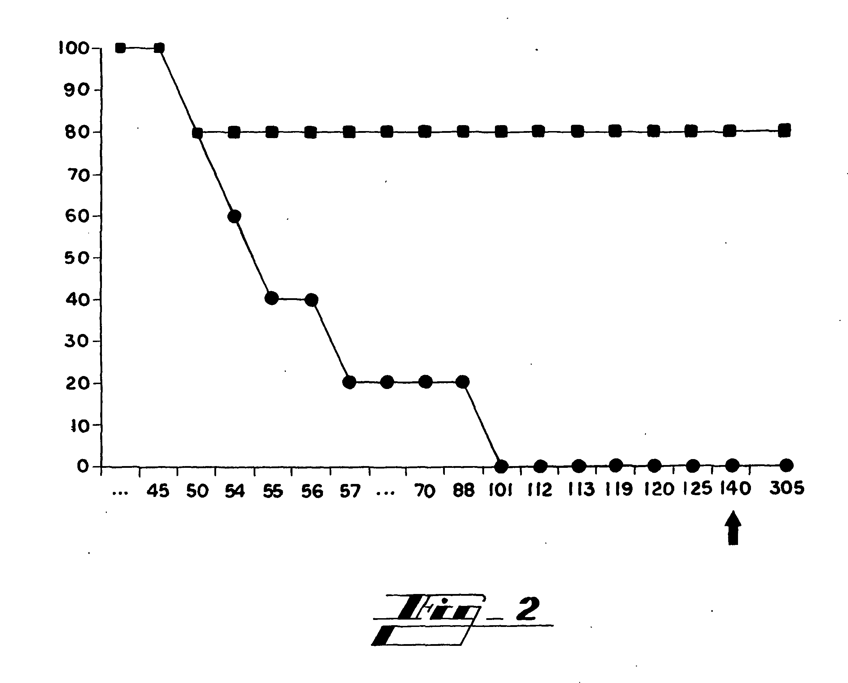

Fig. 2 presents survival curves for female mammals immunized with a composition of the present invention and challenged intravenously with melanoma cells and for female control mammals equally challenged but not immunized. The y-axis represents percentage of survivors and the x-axis represents the number of days of survival. The blackened circles denote female controls and the blackened squares denote female immunized mammals.

### DESCRIPTION OF SPECIFIC EMBODIMENTS

### INTRODUCTION

Group B β-hemolytic *Streptococci* (GBS) are ubiquitous microorganisms.that are generally harmless to humans with one exception - newborn infants infected with GBS frequently develop GBS pneumonia (also called "early onset disease" or "early onset septicemia"), a disease that is associated with high morbidity and mortality. Hellerqvist and colleagues identified a polysaccharide GBS toxin that is a major factor in the complications of GBS pneumonia (Hellerqvist, C.G. et al., Pediatr. Res., 12:892-898 (1981); Sundell, H. et al., J. Pediatr. 137: 338-344 (2000)).

GBS toxin was subsequently found to have many therapeutic properties. It is an anticancer agent that inhibits vascularization of solid tumors (U.S. Patent No. 5,010,062 and corresponding European Patent No. EP 0 445 280 B1; DeVore et al. (1997) Clinical Cancer Res. 3,365-372)). In addition, as described in U.S. Patent No. 5,858,991 and WO98/32453, GBS toxin facilitates wound healing in mammals by minimizing scarring and accelerating healing, and reduces wound-related tumor progression. GBS toxin also enhances repair of neural injuries by minimizing the formation of glial scars (U.S. Patent No. 5,981,508 and WO98/32448) and ameliorates the symptoms of certain chronic inflammatory diseases such as rheumatoid arthritis and psoriasis (WO98/32452).

GBS toxin's anticancer effect has been traced to its ability to rapidly bind tumor-associated endothelial cells and to subsequently activate complement by the alternate (C3) pathway. Activated leukocytes soon infiltrate the endothelial cells, which are subsequently destroyed, and the tumors shrink as a result of the inflammatory response and insufficient blood supply (Van et al., Angiogenesis 2:219-233 (1998); Wamil, B.D. et al., AACR Proceedings, 38:237 (1997)).

Without limitation to a particular theory, it is believed that GBS toxin's other therapeutic effects are due to a similar mechanism. Nerve trauma, wounds, disruption of blood flow, reperfusion, atherosclerotic plaques, rheumatoid arthritis and psoriasis all induce hypoxia which in turn causes the release of vascular endothelial growth factor (VEGF) (Liu et al., J. Neuroscience 17:5395-5406 (1997)). VEGF stimulates endothelial cells to dedifferentiate and begin forming new vasculature by a process known as pathological angiogenesis and also known as pathoangiogenesis. In patients with neural injuries, the newly formed vasculature facilitates gliosis, a proliferation of glial cells which gives rise to glial scars that sterically interfere with reestablishment of neuronal connectivity. Neovasculature in the joints of rheumatoid arthritis sufferers facilitates synovial tissue hyperplasia, pannus formation, and cartilage destruction. Similarly, pathological angiogenesis facilitates the establishment, maintenance and enlargement of psoriatic lesions and is a driving force in the formation of granulation tissue, which leads to chronic wounds or scar formation (including keloids) in the vicinity of wounds. In atherosclerosis, the pathological neovasculature provides oxygen and nutrients to smooth muscle and endothelial cells located below plaques and results in further narrowing of affected blood vessels. Other conditions caused by pathological angiogenesis include diabetic retinopathy, retrolental fibroplasia, neovascular glaucoma, angiofibromas, immune and non-immune inflammation, hemangiomas, Kaposis' sarcoma, and endometriosis (see PCT Publication Number WO 91/10424, page 13, lines 14-21). Additional conditions caused by pathological angiogenesis include corneal graft neovascularization, ocular tumors, trachoma, and hemophiliac joints (see European Patent Application Publication Number EP 0 325 199 A2, page 2, lines 12-19). Additional conditions cuased by pathological angiogenesis include reinopathy of prematurity, macular degeneration, Behcet's syndrome, Osler-Weber-Rendu disease, osteoarthritis, corneal graft rejection and ocular neovascular diseases (see PCT Publication Number WO 94/20085, page 2, line 27 to page 6, line 14). GBS toxin interferes with these harmful processes by binding to the budding pathological neovasculature and targeting it for destruction by the immune system.

It was believed that GBS toxin attacks the lungs of human neonates and binds embryonic neovasculature via receptors present on these tissues at birth and for a short time (about 7 days in term babies and longer in premature infants) thereafter. It was further hypothesized that the same receptors are present later in life only upon pathological neovasculature (i.e. new capillaries formed by pathological angiogenesis). This belief has been confirmed by the recent identification of novel proteins found on such cells that specifically bind GBS toxin. The nucleic acid and amino acid sequences of the human GBS toxin receptor known as HP59 are shown in SEQ ID NO:1 and SEQ ID NO:2, respectively (GenBank Accession Number AF244578). The nucleic acid and amino acid sequences of the sheep GBS toxin receptor known as SP55 (cloned from a sheep lung library, GenBank Accession Number AF244578) are shown in SEQ ID NO:3 and SEQ ID NO:4, respectively. Both HP59 and SP55 are integral proteins with multiple transmembrane domains. Each has several putative sites for phosphorylation by cAMP-dependent kinase, protein kinase C (PKC) and casein kinase II (CK2) as well as putative sites for glycosylation and myristylation. Although HP59 has 41 amino acids at its amino terminus that SP55 lacks, the two proteins are otherwise 87% identical.

As expected, GBS toxin receptor is expressed on the lungs of human neonates and sheep (which are susceptible to infection by GBS). It is not expressed in the vasculature associated with normal (healthy, non-neonate) human ovary, colon, breast or lung tissue, but it is present in the vasculature of tumors in these tissues. Thus, GBS toxin receptor expression is correlated with medical conditions involving pathologic angiogenesis and the receptor is not seen in healthy tissue from humans who are more than one month old (except for premature babies, in which expression is correlated with their due dates).

Although not wishing to be bound by theory, it is believed that since the GBS toxin receptor is present on the pathological neovasculature associated with pathoangiogenic conditions, the immune system, if primed to recognize the GBS toxin receptor, will attack such forming vasculature and the pathoangiogenic condition will be prevented or attenuated. Therefore, the present invention involves methods and compositions for invoking an immune response to the GBS toxin receptor.

### DEFINITIONS

Generally, all technical and scientific terms used herein have the same meaning as commonly understood by one of ordinary skill in the art to which this invention belongs. The nomenclature used herein and the laboratory procedures in immunology, cell culture, biochemistry and molecular biology described below are those well known and commonly employed in the art. Standard techniques are used for recombinant nucleic acid methods, polypeptide synthesis, and production of monoclonal and polyclonal antibodies. Enzymatic reactions and purification steps supplied by manufacturers are typically performed according to the manufacturer's specifications. The techniques and procedures are generally performed according to conventional methods in the art and various general references (*See* generally, Sambrook et al., Molecular Cloning: A Laboratory Manual, 2d ed. (1989) Cold Spring Harbor Laboratory Press, Cold Spring Harbor, N.Y.) which are provided throughout this document. The nomenclature used herein and the laboratory procedures in analytical chemistry, organic synthetic chemistry, immunology and pharmaceutical formulation described below are those well known and commonly employed in the art. Standard techniques can be used for chemical syntheses, chemical analyses, pharmaceutical formulation and delivery, and treatment of mammals. As employed throughout the disclosure, the following terms, unless otherwise indicated, shall be understood to have the following meanings:
For the purposes of this invention, the term "GBS toxin" means any purified faction or component of the natural GBS toxin, or derived from media or protease digests of lysed GBS bacterial, and whose toxicity can be confirmed by either of the following specified assay procedures. The potency of isolated GBS toxin as a tumor growth inhibitor may be ascertained by peroxidase-antiperoxidase (PAP) assays of tumor tissue specimens using anti-GBS toxin IgG, and by infusion in a sheep model at 2 µgs 10⁻¹¹ moles per kg (Hellerqvist, C.G. *et al*., 1981, cited above; Hellerqvist, C.G. et al. PNAS 84:51-56 (1987)).

"GBS toxin receptor" means a proteinaceous molecule capable of binding a toxin from Group B β-hemolytic *Streptococcus* bacteria (GBS toxin). A GBS toxin receptor is usually found in nature on the surface of a cell. Recombinant membrane-bound and soluble GBS toxin receptors can be produced by laboratory techniques known in the art. GBS toxin receptor refers to any receptor that interacts with a GBS toxin as defined herein.

As used herein, a vaccine or method is said to "prevent" or "protect against" a medical condition if its administration to an individual results in the failure of the individual to develop the medical condition.

As used herein, a vaccine or method is said to "ameliorate," "attenuate" or "lessen the severity of" a medical condition if its administration to an individual results either in the suppression or partial suppression of at least one symptom or other manifestation of the medical condition in the individual.

As used herein, a vaccine or method is said to "decrease the incidence of" a medical condition if its administration to an individual has the effect of decreasing the probability that the individual will develop the medical condition.

A "pathoangiogenic condition" is any medical condition associated with pathological angiogenesis such as that caused by for instance the hypoxia-induced, VEGF-mediated formation of new vasculature from dedifferentiated endothelial cells. Pathological angiogenesis occurs in such diseases as diabetic retinopathy, hemangioma, cancer, psoriasis, RA, osteoarthritis and atherosclerosis. (Folkman et al., Science, 235:442 (1987); Kimball et al., Agents & Actions, 34:329 (1991)). It is also is associated with the formation of glial scars in the vicinity of injured nerves and in the formation of keloids, reperfusion injuries and scars during wound healing. Pathological angiogenesis is distinct from physiological neovascularization, a basic repair mechanism that occurs under normal, healthy circumstances (such as wound healing, the female menstrual cycle, and pregnancy) and is believed to result from the proliferation of *existing* endothelial cells after the disruption of contact inhibition (Brem & Folkman, J. Ped. Surg., 28:445 (1993)).

An "immune response" may be humoral or cell-mediated or both. In a humoral response, the immunized animal produces antibodies that recognize and specifically bind a particular antigen. Preferably, the titer: for such antibodies would be at least about 1:200 as measured by ELISA. A cell-mediated immune response is characterized by the presence of helper T cells, suppressor T cells, and/or cytotoxic T cells that recognize the antigen. The presence of such cells can be confirmed by methods known in the art, including the development of a raised area of at least 1mm within 72 hours after the subcutaneous administration of a sample of the antigen.

As used in the context of an antibody administered to a mammal, such an antibody is "immunocompatible" with such mammal if the mammal's immune system does not treat it as a foreign protein and mount an immune response against it. The antibody to be administered may be obtained from a mammal of the same species as the recipient mammal. Alternatively, such antibody can be engineered to contain constant regions that resemble those of antibodies naturally produced by the recipient mammal. Where the recipient mammal is a human, such antibodies are frequently referred to as "humanized" antibodies.

The term "immunogenic" means having antigenic properties or being capable of being specifically bound by an antibody that can specifically bind the antigen. A substance has antigenic properties if it can generate or is capable of eliciting an immune response when administered to an animal under conditions known in the art to facilitate the production of antibodies and/or T cells that will recognize and elicit a cytotoxic response toward cells expressing the particular antigen.

The term "adjuvant" refers to an agent used to enhance the immune response of an immunized mammal to an antigen.

The term "parenteral" as used herein includes subcutaneous injections, intraperitoneal or intramuscular injection, or infusion techniques.

The term "polypeptide" is used herein as a generic term to refer to native protein, fragments, analogs, or glycosylated, phosphorylated or myristylated isoforms of a polypeptide sequence. Hence, native protein, fragments, analogs and glycosylated, phosphorylated and myristylated isoforms are species of the polypeptide genus.

The term "isolated" as used herein in the context of a polypeptide means a polypeptide that is no longer associated with the cell that the polypeptide is normally associated with in nature in the same manner as it is normally associated in nature, such as (1) a polypeptide free of at least some other polypeptides from the same source, (2) a polypeptide expressed by a cell from a different species, (3) a polypeptide that does not occur in nature, and (4) a polypeptide produced from cDNA, recombinant RNA, or synthetic origin or some combination thereof.

The term "naturally occurring", means found in nature. For example, a polypeptide or polynucleotide sequence that is present in an organism (including viruses) found in nature and which has not been intentionally modified in the laboratory is naturally occurring.

As applied to polypeptides, the term "substantial identity" means that two peptide sequences, when optimally aligned by the BLAST computer program, share at least about 80 percent sequence identity, at least about 86 percent sequence identity, and preferably at least about 90 percent sequence identity. Gaps (in either of the two sequences being matched) are allowed in maximizing matching; gap lengths of about 10 or less are preferred, with about 5 or less being more preferred. Residue positions which are not identical may differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine.

The term "fragment" as used herein refers to a peptide that has an amino-terminal, internal and/or carboxy-terminal deletion, but where the remaining amino acid sequence is identical to the corresponding positions in the naturally occurring sequence deduced, for example, from a full-length DNA sequence. Fragments typically are at least about 3 amino acids long, preferably are about 5-10 amino acids long, more preferably are about 10-50 amino acids long, and even more preferably are more than about 50 amino acids long. Also preferred are fragments that comprise one or more extracellular domains of a GBS toxin receptor. Such fragments may also comprise portions of transmembrane and intracellular domains sufficient to maintain the polypeptide fragment in a stereochemical conformation on the surface of a cell, lipid membrane, liposome, micelle, or other lipophilic structure.

The singular forms "a," "an" and "the" include plural referents unless the context clearly dictates otherwise.

The SEQ ID NOs of the nucleic acid and amino acid sequences described herein are summarized below in Table 1.

**Table 1 -- Nucleic Acid and Amino Acid Sequences**

| **SEQ ID NO:** | **Type of Sequence** | **Description** |
|---|---|---|
| SEQ ID NO:1 | Nucleic acid | Full-length human GBS toxin receptor (HP59) |
| SEQ ID NO:2 | Amino acid | Full-length human GBS toxin receptor (HP59) |
| SEQ ID NO:3 | Nucleic acid | Sheep GBS toxin receptor (SP55) |
| SEQ ID NO:4 | Amino acid | Sheep GBS toxin receptor (SP55) |

The headings provided herein describe the general topic discussed and are not intended to be exclusive of information discussed in other sections. Frequently, information, methods, compositions, and other aspects may be applicable to more than one embodiment of the invention and can be so combined.

Disclosed is a method for preventing a pathoangiogenic condition in a mammal by administering to the mammal an amount of one or more GBS toxin receptors or immunogenic fragments thereof effective to induce or maintain in the mammal an immune response to at least one of the GBS toxin receptors.

Disclosed is a method for attenuating a pathoangiogenic condition in a mammal by administering to the mammal an amount of one or more GBS toxin receptors or immunogenic fragments thereof effective to induce or maintain in the mammal an immune response to at least one of the GBS toxin receptors. This method may be performed at a time when the mammal does not have any symptoms of the pathoangiogenic condition. Such administration serves to lessen the severity or progression of the subsequently developed pathoangiogenic condition. When the method is performed on a mammal suffering from the pathoangiogenic condition, it will ameliorate one or more symptoms of the pathoangiogenic condition.

The aforementioned methods may be performed on any animal that expresses a GBS toxin receptor on pathological neovasculature. Preferably, such animal is a mammal that does not express the receptor on healthy tissues. It is known that human embryos and full-term newborn babies up to about 10 days old have GBS toxin receptors on their lung vasculature. Sheep, cows and cats express GBS toxin receptors on their lung vasculature shortly after birth and thereafter. Preferred recipients of this method are full-term humans older than ten days, dogs, mice, pigs, goats and horses.

Immunized animals may receive antibodies to GBS toxin receptor or immunogenic fragments thereof, or expanded autologous T cells to GBS toxin receptor, or combinations thereof. These methods may be used to attack the vasculature in pathoangiogenic conditions. These embodiments may be used independently of each other, or in I combination with the treatments disclosed herein for pathoangiogenic diseases, or in combination with other treatments for pathoangiogenic diseases used in the art.

For example, as stated earlier, it was believed that GBS toxin attacks the lungs of human neonates and binds embryonic neovasculature via receptors present on these tissues at birth. GBS toxin was subsequently found to inhibit vascularization of solid tumors and ameliorate the symptoms of other diseases. Therefore, in another embodiment of the invention, in addition to receiving at least one GBS toxin receptor or at least one immunogenic fragment thereof, immunized animals also receive GBS toxin or fragments thereof, antibodies to at least one GBS toxin receptor or immunogenic fragments thereof, or expanded autologous T cells or combinations thereof. The supplemental treatments are preferred when the animal is at high risk for or is currently suffering from a pathoangiogenic condition. GBS toxin may be obtained from CarboMed, Inc. (Brentwood, TN) or may be purified as taught in U.S. Patent Nos. 5,010,062 and 5, 811,403 and WO98/14603. For human recipients, GBS toxin is preferably administered in amounts in the range of about 5µg/kg to about 25µg/kg. Methods for administering GBS toxin for the treatment of the following pathoangiogenic conditions are taught in the following patents:

| Condition(s) | Patent |
|---|---|
| Cancer | U.S. 5,010,062 |
| Scaring | U.S. 5,858,991 |
| Keloids | U.S. 5,858,991 |
| Reperfusion injury | U.S. 5,858,991 |
| Atherosclerosis | U.S. 5,858,991 |
| Burns | U.S. 5,858,991 |
| Chronic wounds | U.S. 5,858,991 |
| Gliosis | U.S. 5,981,508 |
| Rheumatoid arthritis | WO98/32452 |
| Psoriasis | WO98/3245 |

Antibodies to the GBS toxin receptor can be obtained by immunizing animals including rabbits, mice, goats and chickens with the GBS toxin receptor or an immunogenic fragment thereof . Monoclonal antibodies, polyclonal antibodies and variants thereof can be used. Examples of variants include, but are not limited to, single-chain (recombinant) antibodies, "humanized" chimeric antibodies, and immunologically active fragments of antibodies (e.g., Fab and Fab' fragments). The production of non-human monoclonal antibodies, e.g., murine, is well known (see, e.g., Harlow et al., Antibodies A Laboratory Manual, Cold, Spring Harbor Press, pp. 139-240, 1989). Immunocompatible antibodies are preferred to prevent the immunized animal from mounting an immune response to the GBS toxin receptor antibodies. To prepare antibodies that are immunocompatible to a human, it is desirable to transfer antigen binding regions of non-human monoclonal antibodies, e.g. the F(ab')₂ or hypervariable regions of murine monoclonal antibodies, to human constant regions (Fc) or framework regions by recombinant DNA techniques to produce substantially human molecules. Such methods are generally known and are described in, e.g., U.S. Pat. Nos. 4,816,397 and 4,946,778, and EP publications 173,494 and 239,400. Alternatively, one may isolate DNA sequences which code for a human monoclonal antibody or portions thereof that specifically bind to the receptor protein by screening a DNA library from human B cells according to the general protocol outlined in WO 90/14430, and then cloning and amplifying the sequences which encode the antibody (or binding fragment) of the desired specificity. These sequences may be inserted into the DNA of a mammal in such a manner that the mammal will secrete the antibodies into its milk (Genzyme Transgenics, Framingham, MA). Such antibodies may be administered intraperitoneally or intravenously. Alternate regimens can be determined by one of skill in the art using the basic principles discussed under "Administration" below.

The antibodies can also be used to develop a method of targeting a cytotoxic agent for delivery to a cell that expresses a GBS toxin receptor. For example, a cytotoxic agent can be coupled to an antibody that binds a GBS toxin receptor for selective delivery to the neovasculature of a growing tumor. Such a delivery system would permit a highly concentrated, localized attack on a growing tumor, while minimizing the adverse systemic side effects encountered with most chemotherapeutics.

The sections that follow address in greater detail the selection of immunogenic fragments of the GBS toxin receptor, the production of the receptor polypeptide, identification of other GBS toxin receptors, composition preparations, vaccine preparations, pharmaceutical compositions, administration of the compositions or vaccines, and monitoring of immune response.

### IMMUNOGENIC PEPTIDES OF THE GBS TOXIN RECEPTOR

The administration of one or more immunogenic fragments of a GBS toxin receptor is disclosed. Preferred receptors are HP59 and SP55 and preferred fragments include the Hab1, Hab2 and Hab3 peptides shown in Table 3 and used in Example 1. Preferably the mammal receives a combination of at least two immunogenic fragments and more preferably all three such fragments. Co-administration of fragments from HP59 and SP55 may be desirable to elicit a robust immune response. The immunogenic fragments may contain naturally occurring post-translation modifications such as glycosylation, myristylation or phosphorylation. Other immunogenic fragments of a GBS toxin receptor may be administered and one of skill in the art can readily identify appropriate fragments using techniques known in the art.

A number of methods has been developed to predict the location of immunogenically important epitopes on proteins. The outcome of the combined predictions gives a good forecast of antigenic sites. Suitable GBS toxin receptor fragments may be selected, for instance, from the most hydrophilic parts of the receptor, e.g. by applying the technique described by Hopp and Woods (T. P. Hopp and K. R. Woods (1981): Proc. Natl. Acad. Sci, U.S.A. 78, 3824-3828). Another suitable method for selecting immunogenic fragments is described by Chou and Fasman (P. Y. Chou and G. D. Fasman (1987) Advances in Enzymology 47, 45-148). Various additional algorithms can be used to predict the antigenically important regions of the GBS toxin receptor, such as a prediction of the flexibility of the polypeptide chain (P. A. Karplus and G. E. Schultz, 1985, Naturwissenschaften 72, 212-213), a beta-turn probability profile (P. Y. Chou and G. D. Fasman, 1979, Biophys. J. 26, 367-385), the probability profiles in the 3 conformations for the sequence (Gascuel, O. and J. L. Golmard, 1988, CABIOS 4, 357-365), a prediction of the secondary structure of the sequence (J. Novotny and C. Auffray, 1984, Nucleic Acids Research 12, 243-255). Additional information on the location of relevant epitopes can be obtained using the PEPSCAN-method, developed by Geysen and Meloen (H. M. Geysen, R. H. Meloen and S. J. Barteling (1984) Proc. Natl. Acad. Sci., U.S.A. 81(13); 3998-4002). All of these techniques can be used to select suitable GBS toxin receptor fragments for use as immunogenic antigens.

In addition, immunoreactive epitopes of the GBS toxin receptor can be identified by expressing DNA fragments from the GBS toxin receptor gene in suitable plasmids, such as the pEX plasmids (K. Stanley and J. P. Luzio, 1984. EMBO J. 3, 1429-1434, and J. G. Kusters, E. J. Jager and B. A. M. Van der Zeijst, 1989. Nucl. Acids Res., 17, 8007). In this system, heterologous expression leads to the synthesis of a C-terminal extension of the cro-β-galactosidase hybrid protein. Restriction-endonuclease sites in the GBS toxin receptor DNA sequence can be used to obtain fragments of the GBS toxin receptor gene for insertion into the pEX plasmids. pEX clones synthesizing fusion proteins derived from different overlapping regions of the GBS toxin receptor are then used for further characterization. The GBS toxin receptor fragments are purified, fractionated by polyacrylamide gel electrophoresis, and blotted to nitrocellulose membranes. These membranes are then reacted with polyclonal antibodies directed at the GBS toxin receptor. Only the fragments containing the immuno-reactive epitopes will react with these antibodies. To delineate the minimal length of the epitopes, the DNA inserts of the reactive clones can be progressively shortened by Exonuclease III digestion, or by cloning synthetic oligonucleotides encoding small overlapping parts of the GBS toxin receptor (J. G. Kusters, E. J. Jager, G. Koch, J. A. Lenstra. W. P. A. Posthumus, R. H. Meloen and B. A. M. Van der Zeijst, 1989. J. Immunol., 143, 2692-2698). The epitopes can then be tested for their abilities to generate an immune response in the mammal of choice. Such testing can be performed using methods known in the art as well as methods described in the "Monitoring Immune Response" section of this application.

Using the method of Hopp and Woods via the "Antigen" program in PC/GENE, Hellerqvist and colleagues have identified three regions of SP55, shown below in Table 2, as having high hydrophilicity and as likely to be immunogenic.

**Table 2 - High Points of Hydrophilicity in SP55**

| No. | Average Hydrophilicity | Sequence |
|---|---|---|
| 1 | 2.05 | Glu-Glu-Gly-Ser-Asp-Arg (residues 14-19 of SEQ ID NO:4) |
| 2 | 1.52 | Lys-Asp-Asn-Arg-Thr-Ser (residues 75-80 of SEQ ID NO:4) |
| 3 | 1.33 | Arg-Ala-Pro-Arg-Ala-Glu (residues 25-30 of SEQ ID NO:4) |

Hellerqvist and colleagues have successfully prepared mouse monoclonal antibodies directed to the portions of HP59 shown in Table 3 and rabbit polyclonal antibodies directed at the peptides from SP55 shown in Table 4. The rabbit polyclonal antibodies bind both SP55 and HP59 and several of the hybridomas produce monoclonal antibodies that recognize both SP55 and HP59.

**Table 3 - Immunogenic Peptides from HP59**

| **Peptide** | **Amino Acid Sequence** | **Size** | **SEQ ID Ref.** |
|---|---|---|---|
| Hab1 | LARIVDGEESTDRTPL | 15 aa | residues 49-63 of SEQ ID NO:2 |
| Hab2 | NTTLEDNRTSKACP | 14 aa | residues 112-125 of SEQ ID NO:2 |
| Hab3 | PPRPVQPARPGGFGLSGRRSL | 21 aa | residues 8-28 of SEQ ID NO:2 |
| Hab4 | LARNDGEESTDRTPLLPGAPR AEAAPVC | 28 aa | residues 49-76 of SEQ ID NO:2 |

**Table 4 - Immunogenic Peptides from SP55**

| **Peptide** | **Amino Acid Sequence** | **Size** | **SEQ ID Ref.** |
|---|---|---|---|
| p56a | APSDGEEGSDRTPLLQRAPRAEPAPVC | 27 aa | residues 9-35 of SEQ ID NO:4 |
| p55a¹ | LAPSDGEEGSDRTPL | 15 aa | residues 8-22 of SEQ ID NO:4 |
| p57a² | NTTAKDNRTSYECA | 14 aa | residues 71-84 of SEQ ID NO:4 |

| | | | |
|---|---|---|---|
| ¹Peptide p55a is a fragment of an extracellular domain of GBS toxin receptor. ²Peptide p57a is a fragment of an intracellular domain of GBS toxin receptor. | | | |

### PREPARATION OF GBS TOXIN RECEPTOR POLYPEPTIDES

The GBS toxin receptor polypeptides may be utilized in an unmodified state. Alternatively, the polypeptides may be glycosylated, myristylated, or phosphorylated at one or more of the putative sites for such post-translational modifications. The GBS toxin receptor protein or polypeptides may be prepared as homopolymers (a multitude of identical GBS toxin receptor polypeptides coupled) or heteropolymers (one or more GBS toxin receptor polypeptides coupled to one or more different GBS toxin receptor polypeptides), or may be coupled to one or more other compounds in order to enhance immunogenicity.

In one embodiment the GBS toxin receptor protein is naturally occurring and can be isolated from a cell extract by protein purification techniques known in the art, such as, for example, ion exchange column chromatography, high performance liquid chromatography (HPLC), reverse phase HPLC, or affinity chromatography using antibodies that recognize the GBS toxin receptor. The purified protein can be optionally lyophilized and stabilized.

In another embodiment, the GBS toxin receptor or polypeptide fragments from it can be synthesized chemically by techniques well known in the art, such as solid-phase peptide synthesis (Stewart et al., SOLID PHASE PEPTIDE SYNTHESIS, W.H. Freeman Co., San Francisco (1963)); Merrifield, J. Am. Chem. Soc. 85:2149-2154 (1963)). These and other methods of peptide synthesis are also exemplified by U.S. Patent Nos. 3,862,925, 3,842,067, 3,972,859, and 4,105,602. The synthesis can use manual synthesis techniques or automatically employ, for example, an Applied BioSystems 430A or 431A Peptide Synthesizer (Foster City, California) following the instructions provided in the instruction manual supplied by the manufacturer.

Alternatively, the polypeptides can be expressed using polynucleotides encoding the polypeptide(s) in operative association with an appropriate control sequence including a promoter in an expression vector suitable for expression, preferably in a mammalian cell, and also in bacterial, insect, or yeast cells. Preferably, the GBS toxin receptor polynucleotide or a fragment thereof can be expressed in a mammalian system. Such expression will usually depend on a mammalian promoter, which is any DNA sequence capable of binding mammalian RNA polymerase and initiating the downstream (3') transcription of a coding sequence (e.g. structural gene) into mRNA. Usually, a promoter will have a transcription initiation region which is usually placed proximal to the 5' end of the coding sequence. This transcription initiation region typically includes an RNA polymerase binding site and a transcription initiation site.

Vectors suitable for replication in mammalian cells are known in the art, and can include viral replicons, or sequences that ensure integration of the sequence encoding PAK65 into the host genome. Suitable vectors can include, for example, those derived from simian virus SV40, retroviruses, bovine papilloma virus, vaccinia virus, and adenovirus. A suitable vector, for example, is one derived from vaccinia viruses. In this case, the heterologous DNA is inserted into the vaccinia genome. Techniques for the insertion of foreign DNA into the vaccinia virus genome are known in the art, and utilize, for example, homologous recombination. The insertion of the heterologous DNA is generally into a gene that is non-essential in nature, for example, the thymidine kinase gene (tk), which also provides a selectable marker. Plasmid shuttle vectors that greatly facilitate the construction of recombinant viruses have been described (see, for example, Mackett et al. (1984); Chakrabarti et al. (1985); Moss (1987)). Expression of the heterologous polypeptide then occurs in cells or individuals which are immunized with the live recombinant vaccinia virus.

A specific case of the above embodiment is a so-called vector vaccine in which recombinant polynucleotide are produced in the immunized animal via viral vectors. The viruses applicable for this purpose should have the ability to replicate in the animals to be immunized.
These viruses, furthermore, should possess a genomic region suitable for insertion of the GBS toxin receptor protein or polypeptide gene. Suitable viruses for this purpose are for example enteral viruses such as certain adeno viruses. A particular application of the present invention is concerned with bacterial vector vaccines in which bacteria capable of colonizing the mammal (e.g. *Salmonella* bacteria) are transformed in order to enable them to express a GBS toxin receptor polypeptide in such a way that it will lead to an immunogenic response against the GBS toxin receptor.

Suitable mammalian expression vectors usually contain one or more eukaryotic transcription units that are capable of facilitating expression in mammalian cells. The transcription unit is comprised of at least a promoter element to mediate transcription of foreign DNA sequences. Suitable promoters for mammalian cells are known in the art and include viral promoters such as those from simian virus 40 (SV40) (Subramani et al., Mol Cell. Biol. 1:854-864, 1981), cytomegalovirus (CMV) (Boshart et al., Cell 41:521-530, 1985), Rous sarcoma virus (RSV), adenovirus (ADV) (Kaufman and Sharp, Mol. Cell. Biol. 2:1304-1319, 1982), and bovine papilloma virus (BPV), as well as cellular promoters, such as a mouse metallothionein-1 promoter (U.S. Patent No. 4,579,821), a mouse VK promoter (Bergman et al., Proc. Natl. Acad. Sci. USA 81:7041-7045, 1993; Grant et al., Nuc. Acids Res. 15:5496, 1987), and a mouse VH promoter (Loh et al., Cell 33:85-93, 1983).

The optional presence of an enhancer element (enhancer), combined with the promoter elements described herein, will typically increase expression levels. An enhancer is any regulatory DNA sequence that can stimulate transcription up to 1000-fold when linked to endogenous or heterologous promoters, with synthesis beginning at the normal mRNA start site. Enhancers are also active when they are placed upstream or downstream from the transcription initiation site, in either normal or flipped orientation, or at a distance of more than 1000 nucleotides from the promoter (Maniatis et al. (1987) Science 236:1237; Alberts et al. (1989) MOLECULAR BIOLOGY OF THE CELL, 2nd ed.). Enhancer elements derived from viruses can be particularly useful, because they typically have a broader host range. Examples useful in mammalian cells include the SV40 early gene enhancer (Dijkema et al. (1985) EMBO J. 4:761) and the enhancer/promoters derived from the long terminal repeat (LTR) of the Rous Sarcoma Virus (Gorman et al. (1982b) Proc. Natl. Acad. Sci. 79:6777), from human cytomegalovirus (Boshart et al. (1985) Cell 41:521) as well as the mouse µ enhancer (Gillies, Cell 33:717-728, 1983). Additionally, some enhancers are regulatable and become active only in the presence of an inducer, such as a hormone or metal ion (Sassone-Corsi and Borelli (1986) Trends Genet. 2:215; MAniatis et al. (1987) Science 236:1237).

In addition, the transcription unit can also be comprised of a termination sequence and a polyadenylation signal which are operably linked to the GBS toxin receptor coding sequence. Polyadenylation signals include, but are not limited to, the early or late polyadenylation signals from SV40 (Kaufman and Sharp), the polyadenylation signal from the Adenovirus 5 E1B region and the human growth hormone gene terminator (DeNoto et al., Nuc. Acids Res. 9:3719-3730, 1981).

Sequences that cause amplification of the gene may also be desirable, as are sequences which encode selectable markers. Selectable markers for mammalian cells are known in the art, and include, for example, thymidine kinase, dihydrofolate reductase (together with methotrexate as a DHFR amplifier), aminoglycoside phosphotransferase, hygromycin B phosphotransferase, asparagine synthetase, adenosine deaminase, and antibiotic resistant genes such as neomycin.

A GBS toxin receptor, or fragment thereof, can be expressed on the surface of a cell, or can be expressed in soluble or secreted form. Expression on the surface of the cell can be achieved, for example, by including a secretory leader operably linked to a nucleic acid sequence encoding the desired receptor fragment and at least one transmembrane domain. The secretory leader can be that encoded by the GBS toxin receptor gene, or can be a heterologous leader sequence commonly used in the art, such as, for example, the leader sequence of *Schizosaccharomyces pombe* pho1⁺ acid phosphatase (Braspenning et al., Biochem Biophys Res. Commun. (1998) 245:166-71), the leader sequence of human interleukin-2 (IL-2) gene (Sasada et al., Cell Struct Funct (1988) 13:129-141). Expression in soluble or secreted form can be achieved, for example, by excluding from the gene construct nucleic acid sequences encoding a transmembrane domain. In some instances, solubility and/or secretion are achieved by the use of a fusion partner, such as, for example, chloramphenicol acetyltransferase (CAT), β-galactosidase, and other genes readily expressed in the selected host cell.

The vector that encodes GBS toxin receptor can be used for transformation of a suitable mammalian host cell. Transformation can be by any known method for introducing polynucleotides into a host cell, including, for example packaging the polynucleotide in a virus and transducing a host cell with the virus or by transfection procedures known in the art, as exemplified by U.S. Patent Nos. 4,399,216, 4,912,040, 4,740,461, and 4,959,455. The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are known in the art and include many immortalized cell lines available from the American Type Culture Collection (ATCC), including, but not limited to, Chinese hamster ovary (CHO) cells, HeLa cells, baby hamster kidney (BHK) cells, monkey kidney cells (COS), N1E-115 (Liles et al., J. Biol. Chem. 261:5307-5313, 1986), PC 12 human hepatocellular carcinoma cells (e.g., Hep G2), and a number of other cell lines, such as insect derived cell lines IF9 and IF21. Cell lines of particular preference are those expressing recombinant GBS toxin receptor constructs constitutively, lines which subsequently develop characteristics of a transformed cell, and lines that more preferably express GBS toxin receptor or fragments on the cell surface. Particularly preferred are ECV cells (a bladder carcinoma cell line originally referred to in the scientific literature as an endothelial cell line), human umbilical vein endothelial cells (HUVEC), bovine, sheep, and human adrenal medulla endothelial cells.

Recombinant GBS toxin receptor or fragments thereof can be produced by culturing host cells expressing the receptor or fragment in a suitable culture medium and under appropriate cell culture conditions. Culture media and conditions are variable depending on the requirements of a particular host cell line and are well known in the art. Typically, cells are cultured at 37°C in a cell culture incubator with a fixed amount of CO₂, usually in the range of 5-10%.

### IDENTIFICATION OF OTHER GBS TOXIN RECEPTORS

In addition to the preferred GBS toxin receptors HP59 and SP55, other naturally occurring GBS toxin receptors can be used in methods, compositions and vaccines. Nucleic acids encoding such receptors can be isolated from various tissue sources and cell cultures from different species that produce such a receptor by the methods described herein, such as, for example, cells from tumor endothelium, synovial tissue in rheumatoid arthritis, or hypoxic tissue deprived of or restricted from blood flow, such as in reperfusion injury or wounded tissue. Such polynucleotides can be isolated by hybridization using probes or by polymerase chain reaction using oligonucleotides, as well as by implementing other molecular biology techniques known in the art. Such probes and oligonucleotides typically comprise various regions of the sequence of SEQ ID NO:1 or 3, or encode various regions of the sequence of SEQ ID NO:2 or 4. Alternatively, additional target proteins for CM101 may be expression cloned. This method is described in WO 00/05375. Alternatively, subtractive hybridization between similar tissues that do and do not express the GBS toxin receptor may be used to isolate additional GBS toxin receptors. Examples of such tissues are wounded tissue and corresponding non-wounded tissue; tumor tissue and a sample of the same tissue that is free of histopathological abnormality; and lung tissue of appropriate mammals during expression of the GBS toxin receptor and shortly after expression stops. Upon discovery of an additional receptor, the probes may comprise various regions of the newly discovered receptor.

Polynucleotides useful for cloning genes encoding GBS toxin receptors of various organisms can be determined by comparing the amino acid sequences of homologous proteins. (see Table 5 which compares amino acids 41-536 of HP59 with amino acids 1-495 of SP55). For example, conserved regions can be targeted for the synthesis of oligonucleotides or degenerate oligonucleotides to be used as probes for hybridization or nucleic acid amplification, techniques discussed further below. Stringency can be varied to achieve selective hybridization conditions whereby nucleic acid sequences having less than 95% identity with respect to each other will hybridize. These conditions are known in the art and discussed herein. Generally, the nucleic acid sequence identity between HP59 or SP55 and a nucleic acid sequence of interest will be at least about 80%, and more typically with preferably increasing identities of at least about 85% and 90%.

Polynucleotides can be used as probes under high stringency wash conditions and with corresponding hybridization conditions, as known in the art. Small polynucleotides, for example, polynucleotides 200 bases or fewer in length, are often referred to in the art as oligonucleotides. Techniques for using polynucleotides as probes to detect the same or related nucleic acid sequences is well known in the art. See, for example, Sambrook *et al*, especially Chapter 11. Usually, probes can be made from polynucleotides that are 10 to 200 bases in length. Preferably probes are made from polynucleotides 10 to 60 nucleotides in length and most preferably 12 to 40 bases in length. Specific probes can be designed based on results obtained using nucleic acid homology computer programs such as FASTA, which uses the method of Pearson and Lipman (Proc. Natl. Acad. Sci. USA 85:2444-2448 (1988)) and shows the degree of identity between compared sequences. The size of the probe is dependent upon the region of the gene to which it will be hybridized. The size of the probe increases as the degree of homology to undesirable nucleic acid sequences increases. A probe 10-50 nucleotides in length can be used, preferably more than 50 nucleotides, even more preferably more than 100 nucleotides, and most preferably a probe made from the entire coding region of a GBS toxin receptor will be used. To decrease the number of false positives, preferably two probes are used to identify clones that bind to both probes under hybridization and wash conditions. Oligonucleotides can be synthesized on an Applied BioSystems oligonucleotide synthesizer according to specifications provided by the manufacturer.

Typically, hybridization and washing conditions are performed at according to conventional hybridization procedures. Typical hybridization conditions for screening plaque lifts (Benton and Davis (1978) Science 196: 180) can be: 50% formamide, 5 x SSC (sodium chloride, sodium citrate) or SSPE (sodium chloride, sodium phosphate, EDTA), 1-5 x Denhardt's solution, 0.1-1% SDS, 100-200 µg sheared heterologous DNA or tRNA, 0-10% dextran sulfate, 1 x 10¹ to 1 x 10⁷ cpm/ml of denatured probe with a specific activity of about 1 x 10⁸ cpm/µg, and incubation at 42°C for about 6-36 hours. Prehybridization conditions are essentially identical except that probe is not included and incubation time is typically reduced. Washing conditions are typically 1-3 x SSC, 0.1-1% SDS, 42-70°C with change of wash solution at about 5-30 minutes. For high stringency hybridization conditions, various parameters can be altered to increase the stringency of hybridization, such as by increasing the temperature of incubation with the labeled probe. Preferably, for greater flexibility in experimental design, the probe can be hybridized at a lower temperature, such as, for example, room temperature and the stringency can then be modified by altering the salt concentration and temperature of the wash solutions. For high stringency a wash temperature of greater than or equal to 42°C can be used, such as, for example, 68°C, in a wash buffer having a salt concentration less than 3X SSC, such as, for example, 0.1X SSC. In some cases, TMACL can also be used, particularly for polynucleotides rich in G-C base pairs in order to decrease non-specific binding. A lower stringency wash can be used to hybridize polynucleotides with lower identities or polynucleotides that are less than 60 base pairs in length. For a low stringency wash, temperatures of less than or equal to 42° can be used in a wash buffer having a salt concentration of greater than or equal to 2X SSC.

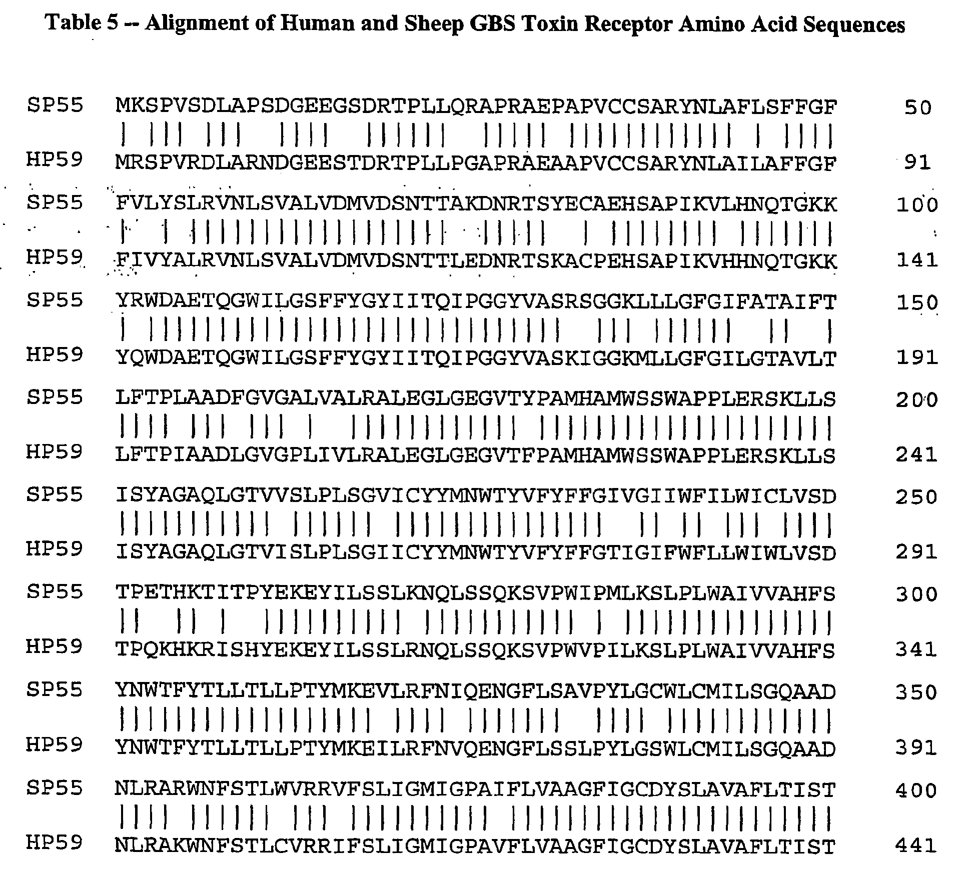

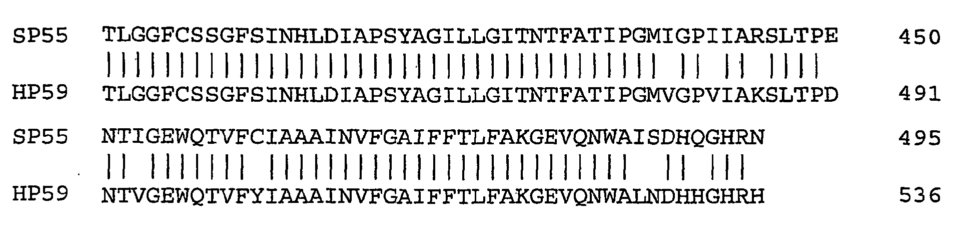

### VACCINE PREPARATION

A preferred embodiment is a conjugate vaccine in which the GBS toxin receptor or fragment thereof is covalently linked to a "carrier" protein or polypeptide. The linkage serves to increase the antigenicity of the GBS toxin receptor polypeptide. Methods for forming conjugate vaccines from an antigenic molecule and a "carrier" protein or polypeptide are known in the art (Jacob, C. O, et al., Eur. J. Immunol. 16:1057-1062 (1986); Parker, J. M. R. et al., In: MODERN APPROACHES TO VACCINES, Chanock, R. M. et al., eds, pp. 133-138, Cold Spring Harbor Laboratory, Cold Spring Harbor, N.Y. (1983); Zurawski, V. R, et al., J. Immunol. 121:122-129 (1978); Klipstein, F. A, et al., Infect. Immun. 37:550-557 (1982); Bessler, W. G, Immunobiol. 170:239-244 (1985); Posnett, D. N, et al., J. Biol. Chem. 263:1719-1725 (1988); Ghose, A. C, et al., Molec. Immunol. 25:223-230 (1988)). A prototype model for conjugate vaccines was developed against *Hemophilus influenzae* (Anderson, P, Infec. and Immun. 39:223-238 (1983); Chu, C, et al., Infect. Immun. 40:245-256 (1983); Lepow, M, Pediat. Infect. Dis. J. 6:804-807 (1987)), and this model may be employed in constructing the novel vaccines of the present invention. Additional methods for producing such a conjugate vaccine are disclosed by Anderson, P. W, *et al*., European Patent Publication 245,045; Anderson, P. W, et al., U.S. Pat. Nos. 4,673,574 and 4,761,283; Frank, R. et al., U.S. Pat. No. 4,789,735; European Patent Publication No. 206,852; Gordon, L. K, U.S. Pat. No. 4,619,828; and Beachey, E. H, U.S. Pat. No. 4,284,537. Useful carriers include keyhole limpet hemocyanin (KLH), bovine serum albumin (BSA), ovalbumin, human serum albumin, human gamma globulin, chicken immunoglobulin G, bovine gamma globulin and tetanus toxoid. Alternatively, the GBS toxin polypeptide may be conjugated or linked to immunogenic polysaccharides such as group A *Streptococci* polysaccharide, C-polysaccharide from group B *Streptococci,* or the capsular polysaccharide of *Streptococci pnuemoniae*.

Another embodiment is a whole cell vaccine in which the transfected or normal cell expressing. GBS toxin receptor on its membrane may be alive or fixed and killed.

As would be understood by one of ordinary skill in the art, when the vaccine of the present invention is provided to an individual, it may be in a composition which may contain salts, buffers, adjuvants, or other substances which are desirable for improving the efficacy of the composition. Normally, the adjuvant and the composition are mixed prior to presentation to the immune system, or presented separately, but into the same site of the mammal being immunized. Alum is a registered adjuvant for human use. Other adjuvants are being developed for human use and it is anticipated that these other adjuvants would be suitable for use in preparing compositions for human vaccination in accordance with this invention. Suitable adjuvants for the vaccination of animals include but are not limited to oil emulsions such a Freund's complete or incomplete adjuvant (not suitable for livestock use), Marcol 52: Montanide 888 (Marcol is a Trademark of Exxon. Montanide is a Trademark of SEPPIC, Paris), squalane or squalene, Adjuvant 65 (containing peanut oil, mannide monooleate and aluminium monostearate), mineral gels such as aluminium hydroxide, aluminium phosphate, calcium phosphate and alum, surfactants such a hexadecylamine, octadecylamine, lysolecithin, dimethyldioctadecylammonium bromide, N,N-dioctadecyl-N',N'-bis(2-hydroxyethyl) propanediamine, methoxyhexadecylglycerol and pluronic polyols, polyanions such as pyran, dextran sulfate, polyacrylic acid and carbopol, peptides and amino acids such as muramyl dipeptide, dimethylglycine, tuftsin and trehalose dimycolate.

The polypeptides of the present invention can also be administered following incorporation into liposomes or other micro-carriers.

### PHARMACEUTICAL COMPOSITIONS

Pharmaceutical compositions of the invention preferably include a pharmaceutically acceptable carrier that may contain a variety of components that provide a variety of functions, including regulation of drug concentration, regulation of solubility, chemical stabilization, regulation of viscosity, absorption enhancement, regulation of pH, and the like. For example, in water soluble formulations the pharmaceutical composition preferably includes a buffer such as a phosphate buffer, or other organic acid salt, preferably at a pH of between about 7 and 8. Other components may include antioxidants, such as ascorbic acid, hydrophilic polymers, such as, monosaccharides, disaccharides, and other carbohydrates including cellulose or its derivatives, dextrins, chelating agents, such as EDTA, and like components well known to those in the pharmaceutical sciences, e.g. REMINGTON'S PHARMACEUTICAL SCIENCE, 19th edition (Mack Publishing Company, Easton, PA).

For purposes of parenteral administration, solutions in sesame or peanut oil or in aqueous propylene glycol can be employed, as well as sterile aqueous solutions of the corresponding water-soluble, alkali metal or alkaline-earth metal salts previously enumerated. Such aqueous solutions should be suitably buffered, if necessary, and the liquid diluent first rendered isotonic with sufficient saline or glucose. Solutions of the GBS toxin receptor polypeptide as a free base or a pharmacologically acceptable salt can be prepared in water suitably mixed with a surfactant such as hydroxypropylcellulose. A dispersion can also be prepared in glycerol, liquid polyethylene glycols and mixtures thereof and in oils. Under ordinary conditions of storage and use, these preparations contain a preservative to prevent the growth of microorganisms. These particular aqueous solutions are especially suitable for intravenous, intramuscular, subcutaneous and intraperitoneal injection purposes. In this connection, the sterile aqueous media employed are all readily obtainable by standard techniques well-known to those skilled in the art.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonged absorption of the injectable compositions can be brought about by use of agents delaying absorption, for example, aluminum monostearate and gelatin.

Sterile injectable solutions are prepared by incorporating the GBS toxin receptor polypeptide in the required amount in the appropriate solvent with various of the other ingredients enumerated above, as required, followed by filtered sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique which yield a powder of the active ingredient plus any additional desired ingredient from the previously sterile-filtered solution thereof.

Intranasal formulations may include vehicles that neither cause irritation to the nasal mucosa nor significantly disturb ciliary function. Diluents such as water, aqueous saline or other known substances can be employed with the subject invention. The nasal formulations may also contain preservatives such as, but not limited to, chlorobutanol and benzalkonium chloride. A surfactant may be present to enhance absorption of the subject proteins by the nasal mucosa.

Oral liquid preparations may be in the form of, for example, aqueous or oily suspension, solutions, emulsions, syrups or elixirs, or may be presented dry in tablet form or a product for reconstitution with water or other suitable vehicle before use. Such liquid preparations may contain conventional additives such as suspending agents, emulsifying agents, non-aqueous vehicles (which may include edible oils), or preservative.

### ADMINISTRATION

### ROUTE

The polypeptides, compositions and vaccines of the present invention can be administered orally, parenterally by injection, rapid infusion, nasopharyngeal absorption (intranasopharangeally), dermoabsorption, or rectally. They may alternatively be administered intramuscularly, or intravenously. Compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption. Liquid dosage forms for oral administration may generally comprise a liposome solution containing the liquid dosage form. Suitable forms for suspending liposomes include emulsions, suspensions, solutions, syrups, and elixirs containing inert diluents commonly used in the art, such as purified water. Besides the inert diluents, such compositions can also include adjuvants, wetting agents, emulsifying and suspending agents, and the like.

### DOSAGE AND FREQUENCY OF ADMINISTRATION

The amount of GBS toxin receptor polypeptide to be combined with carrier, diluent, excipient and/or adjuvant to produce a single pharmaceutical composition or vaccine dosage form will vary depending upon the pathoangiogenic condition being vaccinated against, the mammal's propensity for developing such condition or the severity of such condition, the mammal to be treated and the particular time and route of administration. It will be understood, also, that the specific dose level for any particular mammal will depend upon a variety of factors including the age, body, weight, general health, sex, and diet of such mammal. Effective dosages can be readily established by one of ordinary skill in the art through routine trials establishing dose-response curves.

Many different techniques exist for the timing of the immunizations when a multiple administration regimen is utilized. It is possible to use the compositions of the invention more than once to increase the level, diversity and stability of the immune response in the immunized animal. Multiple doses may be required to maintain a state of immunity to GBS toxin receptor. If so, multiple immunizations will be given at intervals appropriate to maintain the desired immune response.

### MONITORING IMMUNE RESPONSE

It is desirable to characterize the immune response of a mammal that has received a vaccine according to the method of the present invention. Such characterization is preferably made in light of the mammal's immune response to the GBS toxin receptor and to various other standards prior to the initial administration of the receptor. Subsequent testing is performed within a reasonable period, preferably at least six weeks, after administration of the GBS toxin receptor. Such testing may be performed again at regular intervals thereafter or on an as-needed basis, depending on the mammal's condition. For instance, at the desired timepoint, a blood sample is harvested from the mammal and preserved in an appropriate manner until testing can be carried out. Preservation methods of the blood samples include separation of blood sera from other blood components, cooling or freezing, and preservation with various buffering or anticoagulatory agents. Testing may also be performed on urine, stool, and tissue from lung, lymph nodes, spleen, bond marrow, ulcers, skin lesions and exudate and other tissues affected by the pathoangiogenic condition being tested.

The presence of antibodies in the serum (i.e. a humoral immune response to the GBS toxin receptor) may be determined by ELISA, RAST, EIA, hemagglutination inhibition, other radioimmunoassay techniques, and latex agglutination methods and by general serological assays including white blood cell counts, measurement of total and differentiated immunoglobulin levels, including IgM, total IgG, IgG₁, IgG₂, IgG₃ and IgG₄, or other methods known to one of skill in the art. One possible method for performing an ELISA determination involves plating the GBS toxin receptor of interest onto ELISA plates. After unbound antigen is washed off the plates, excess protein reactive sites on the plates are blocked with a blocking buffer. Following removal of the blocking buffer, a dilution series of sera in PBS is added into wells of the blocked ELISA plates and incubated for one hour at 37°C. After removal of the dilution series, the plates are washed and a conjugated antibody solution in PBS (antibody is against the vaccinated species) is added to the wells of the ELISA plate, which is then incubated for one hour at 37°C. After removal of the conjugated antibody solution, the plates are washed and the appropriate chromogenic substrate is added to the wells to allow for color development. After a 30-minute incubation, color development is stopped and the absorbance readings of the wells are read on a spectrophotometer. The absorbance readings for each group at each dilution are averaged and plotted versus the reciprocal dilution. Positive and negative controls are an important part of this determination as is a comparison with the pre-immunization (baseline) testing. If antibodies can be detected at a dilution of about 1:200 or more, then the mammal has a positive immune response.

Determination of a cellular immune response may be made by methods known to those of skill in the art, including those discussed herein. The presence of T cells that recognize the GBS toxin receptor can be tested by administering a sample of the GBS toxin receptor subcutaneously and examining the skin of the mammal (preferably human) 72 hours later to see if the skin at the site of administration is raised by about 1mm or more. The size of such wheals are typically measured and can be correlated to the degree of immune response. Such a response is a positive immune response. Alternatively, a cellular immune response can be determined by such methods as measuring C-reactive protein serum levels, complement levels, including normal complement and C-3 detection, erythrocyte sedimentation rate, haptoglobin serum levels, immunoprotein levels and other protein serum levels.

A positive immune response means that the pathoangiogenic condition is prevented or attenuated. Prevention and attenuation can be demonstrated by methods known in the art, including but not limited to those discussed herein.

One method of demonstrating prevention is to administer the vaccine to or practice the method upon a group of individuals from a population that is susceptible to the pathoangiogenic condition of interest and compare the incidence of the condition in such group with the incidence of the condition in the rest of the population that did not receive the vaccine or method. For example, humans who smoke are susceptible to lung cancer. A group from a test population of statistically matched smokers who do not have lung cancer can be given a vaccine or method of the present invention while the remainder of the test population will not receive any treatment. Both groups are monitored for lung cancer. If no members of the group that received the vaccine or method have developed lung cancer by a time when a statistically significant number of the remainder of the test population have developed the disease, then the vaccine or method can be concluded to prevent lung cancer. If cases of lung cancer develop in the treated group but do so at a statistically significant lower rate than that of the remainder of the population, the vaccine or method can be concluded to decrease the incidence of lung cancer. One of skill in the art can demonstrate prevention of other pathoangiogenic conditions by similar methods known in the art including testing of populations that are susceptible to the particular disease on account of genetic and/or environmental factors.

Support for additional aspects of the present invention and methods for making and using the invention are published in WO 00/05375.

### EXAMPLES

### Example 1. Immunization with a mixture of three fragments from human GBS toxin receptor retards tumor growth.

Peptides Hab1, Hab2 and Hab3, which are shown in Table 3, are fragments from amino terminus of HP59 that were selected as immunogens based on hydrophilicity. They were synthesized *in vitro* (Sigma-Genosis, The Woodlands, Texas) and conjugated to keyhole limpet hemocyanin (KLH), a glycoprotein which served as an adjuvant. Experimental C57 mice (n=8, four males and four females) were immunized by subcutaneous injection of 100 micrograms of a mixture of the three peptide conjugates in complete Freund's adjuvant (CFA). Two weeks, four weeks and six weeks later, each of these mice received a subcutaneous injection of 100 micrograms of this mixture in incomplete Freund's adjuvant (IFA). A final injection of 100 micrograms of this mixture in IFA was given intradermally at eight weeks. Control C57 mice (n=8) were immunized at the same times in the same manner with only KLH and Freund's adjuvant.

Experimental mice were bled from the tail vein 6 weeks after the initial immunization and every four weeks thereafter and the level of antibodies formed to the HP59 derived peptides were established by ELISA. When a positive antibody titer was obtained from all experimental mice, both control and experimental mice were challenged with tumor cells as follows: 50,000 mouse melanoma B1-6 cells or Lewis lung tumor cells were suspended in 0.6% agar and injected subdermally. The volume of the tumors was measured 6, 8, 10, 12 and 14 days after the tumor cell injections. As shown in Table 6, the melanoma tumors of immunized mice were 45% smaller than those of their control counterparts and the Lewis lung tumors of immunized mice were 38% smaller than those of their control counterparts. The results of both experiments were statistically significant. For the melanoma experiments, the paired t test for treated versus control was t=2.3898 with five degrees of freedom and the one tailed p=0.0312, which is considered significant. For the Lewis lung experiments, the paired t test for treated versus control was t=2.9899 with five degrees of freedom and the one tailed p=0.0152, which is considered significant.

**TABLE 6. Tumor progression (volume = # x mm³)**

| **Tumor Type** | **Day 6** | **Day 8** | **Day 10** | **Day 12** | **Day 14** |
|---|---|---|---|---|---|
| Melanoma (n=4) | 15.6 | 83.3 | 131.3 | 350.1 | 429.8 |
| Control (n=4) | 20.4 | 118.2 | 174.5 | 518.6 | 783.5 |
| Lewis Lung (n=4) | | 36.9 | 70.8 | 144.8 | 194.6 |
| Control (n=4) | | 68.8 | 119.4 | 178.8 | 315.1 |

### Example 2. Treatment of immunized mammals with CM101

Mice immunized and challenged with tumor cells as described in Example 1 may additionally receive weekly intravenous infusions of 60µg/kg of the GBS toxin CM101 (CarboMed, Inc., Brentwood, TN) or mock injections. Tumor progression is compared in the CM101-treated and control mice and the degree of tumor growth retardation or elimination is calculated using standard statistical methods. Additional experiments at different dose levels are conducted to determine a dose-response relationship.

### Example 3. Treatment of immunized mammals with immunocompatible antibodies

Mice immunized and challenged with tumor cells as described in Example 1 may additionally receive weekly injections of 100µg of a mixture of mouse monoclonal antibodies specific for HP59 or mock injections. Such antibodies are obtained by immunizing mice with 100mg of the synthetic peptides shown in Table 3 in accordance with the methods taught in Harlow et al., ANTIBODIES: A LABORATORY MANUAL, Cold Spring Harbor Press, pp.139-240 (1989). Tumor progression is compared in the antibody-treated and control mice and the degree of tumor growth retardation or elimination is calculated using standard statistical methods. Additional experiments at different dose levels are conducted to determine a dose-response relationship.

### Example 4. Treatment of immunized mammals with autologous T cells

T cells may be removed from mice immunized and challenged with tumor cells as described in Example 1 and either incubated with a mixture of the Hab1, Hab2 and Hab3 peptides or mock incubated for 72 hours. The T cells are returned to each donor mouse by intravenous injection. Tumor progression is compared in the peptide-incubated and control mice and the degree of tumor growth retardation or elimination is calculated using standard statistical methods. Additional experiments at different dose levels are conducted to determine a dose-response relationship.

### Example 5. Immunization with a mixture five fragments from human and sheep GBS toxin receptors retards tumor growth

Two groups of male and female C57 mice and equal size controls were immunized with a mixture of five peptides shown in Tables 3 and 4, p56a, p55a, p57a, Hab1, and Hab2. These peptides were derived from the homologous proteins HP59 and SP55 (87%), conjugated with keyhole limpet hemocyanin (KLH) (Sigma Genosys, TX) and emulsified in complete Freund's Adjuvant (CFA), by intradermal injection in three places at the base of the tail.

The first immunization was followed two weeks and four weeks later with repeat intradermal injections of antigen KLH conjugate and incomplete Freund's Adjuvant (IFA) for the experimental group. IFA alone was given to control animals. Animals were bled after 5 weeks and shown to have antibody titer of 1:200 with optical density (O.D.) of >2.0. to one extracellular peptide based on a seven transmembrane domain (7TMD) configuration.

Lewis Lung cell suspension (5x10⁴ cells) in 3% agar was implanted subcutaneously in seven immunized male and five immunized females. Four male and four female CFA and IFA immunized mice served as controls. The mice were observed until the control tumors began to ulcerate at which time mice were sacrificed and tissues, including tumors, were collected.

The growth curve for the Lewis Lung tumors in C57 mice are shown in Fig. 1.
The y-axis represents tumor volume and the x-axis represents the day the tumor volume was measured. Male controls are denoted by blackened diamonds, female controls are denoted by blackened circles; male immunized are denoted by clear circles and female immunized are denoted by clear diamonds. The raw data of tumor volumes from different days were recorded. A paired t-test using tumor volumes at the five last measurements shows a significant difference (p=0.025) in the average tumor volumes for the non-immunized male and female with the average for the immunized groups. The overall tumor burden in the immunized mice was only 38.3% of the control.

### Example 6. Retardation of melanomas in female mammals

Five each of HP59/CFA and CFA immunized, female C57 mice were inoculated intravenously with 1,000 melanoma cells. Time to death was the endpoint. Mice were to be sacrificed when respiratory distress was obvious.

The five female controls died on days 46, 52, 54, 62 and 100. The autopsy of the diseased control mice showed lungs to be infiltrated by numerous metastasis. One immunized female died early with no sign of tumors. The remaining four immunized mice were all alive at day 140 with no signs of disease, at which time two of them were inoculated with an additional 1,000 melanoma cells (denoted by an arrow in Fig. 2). The remaining four immunized mice continued to show no signs of disease on day 305. Fig. 2 shows survival curves for the female mice challenged intravenously with 1000 melanoma cells. The y-axis represents percentage of survivors and the x-axis represents the number of days of survival. The blackened circles denote female controls and the blackened squares denote female immunized mammals.

## Claims

1. A composition comprising one or more Group B β-hemolytic Streptococci (GBS) toxin receptors or immunogenic fragments thereof and an adjuvant,
wherein at least one immunogenic fragment has substantial identity to the following residues of SEQ ID NO:2: 49 to 63 (Hab1), 112 to 125 (Hab2), or 8 to 28 (Hab3) optionally comprising further
(i) an effective amount of one or more immunocompatible antibodies that bind to a GBS toxin receptor, or
(ii) T cells from a mammal that have been cultured with a GBS toxin receptor, or
(iii) an amount of GBS toxin sufficient to induce a response,
wherein the amount of GBS toxin is for a single dosage of at least about 5 µg/kg body weight.

2. A composition comprising at least one immunogenic fragment of one or more Group B β-hemolytic Streptococci (GBS) toxin receptors
wherein at least one immunogenic fragment has substantial identity to the following residues of SEQ ID NO:2: 49 to 63 (Hab1), 112 to 125 (Hab2), or 8 to 28 (Hab3).

3. The composition of claim 1 or 2, wherein the one or more GBS toxin receptors or immunogenic fragments thereof are in an amount effective for protecting against or attenuating a pathologic angiogenic condition.

4. The composition of any of claims 1 to 3, wherein at least one of the fragments is isolated.

5. The composition of any of claims 1 to 4, further comprising a pharmaceutically acceptable excipient.

6. The composition of any of claims 1 to 5, wherein said adjuvant is selected from the group consisting of a water-in-oil composition, Freund's adjuvant, QS21, IL-12 and interferon gamma.

7. The composition of any of claims 4 to 6, wherein one of the isolated GBS toxin receptors or fragments is conjugated or linked to a protein carrier.

8. The composition of claim 7, wherein the protein carrier is a molecule selected from the group consisting of keyhole limpet hemocyanin (KLH), bovine serum albumin (BSA), ovalbumin, human serum albumin, human gamma globulin, chicken immunoglobulin G, bovine gamma globulin and tetanus toxoid.

9. The composition of any of claims 1 to 8, wherein at least one of the GBS toxin receptors or fragments thereof is glycosylated.

10. The composition of any of claims 1 to 9, wherein at least one isolated GBS toxin receptors or fragments thereof is recombinant or synthetic.

11. The composition of any of claims 1 to 10, wherein at least one GBS toxin receptor has substantial identity to SEQ ID NO:2.

12. The composition of claim 11, wherein at least one GBS toxin receptor is identical to SEQ ID NO:2, or is SEQ ID NO:2 with at least one conservative amino acid substitution.

13. The composition of any of claims 1 to 12, further comprising at least one GBS toxin receptor which has substantial identity to SEQ ID NO:4.

14. The composition of claim 13, wherein the GBS toxin receptor is identical to SEQ ID NO:4, or is SEQ ID NO:4 with at least one conservative amino acid substitution or fragment thereof.

15. The composition of any of claims 1 to 14, wherein at least one immunogenic fragment has substantial identity to a portion of SEQ ID NO:4.

16. The composition of claim 15, wherein at least one immunogenic fragment has substantial identity to the following residues of SEQ ID NO: 4: 8 to 22 (p55a), 9 to 35 (p56a) or 71 to 84 (p57a).

17. The composition of any of claims 1 to 16, wherein each antibody is a monoclonal antibody.

18. The composition of any of claims 1 to 17, wherein each antibody is obtained from a polyclonal serum.

19. The composition of any of claims 1 to 18, wherein at least one of the antibodies further comprises a cytotoxic agent.

20. The composition of any of claims 1 to 19 for use as a vaccine or a medicament.

21. The composition of claim 20 for prevention, attenuation or treatment of a pathologic angiogenic condition which is selected from the group consisting of cancer, scarring during wound healing, gliosis during repair of nerve injury, chronic wounds, keloids, reperfusion injury, rheumatoid arthritis, atherosclerosis, osteoarthritis and psoriasis.

22. The composition of claim 20 or 21 for treatment of a mammal.

23. Use of the composition of any of claims 1 to 22 for the preparation of a vaccine or a medicament for the treatment of a mammal, whereby the normal tissue of the mammal does not contain the GBS toxin receptor.

24. The composition of claim 22 or the use of claim 23, whereby the mammal does not have the pathologic angiogenic condition at the time of the administering step.

25. The composition of claim 22 or the use of claim 23, whereby the mammal has a pathologic angiogenic condition at the time of the administering step.

26. The composition of any of claims 1 to 25, wherein the amount of GBS toxin is for a single dosage of at least about 15 µg/kg body weight.

27. The composition of claim 26, wherein the amount of GBS toxin is for a single dosage of at least about 20 µg/kg body weight.

28. A method for producing a composition of any of claims 1 to 27, comprising:
providing at least one GBS toxin receptor or immunogenic fragment thereof as defined in Claim 1 and formulating the receptor or fragment in a pharmaceutically acceptable excipient and
providing an adjuvant.

## Patentansprüche

1. Eine Zusammensetzung, enthaltend einen oder mehrere Toxinrezeptoren aus β-hämolytischen Streptokokken der Gruppe B (GBS) oder immunogene Fragmente davon und ein Adjuvans,
wobei wenigstens ein immunogenes Fragment im Wesentlichen identisch ist mit den folgenden Resten der SEQ ID Nr. 2: 49 bis 63 (Hab1), 112 bis 125 (Hab2) oder 8 bis 28 (Hab3), gegebenenfalls außerdem enthaltend
(i) eine wirksame Menge eines oder mehrerer immunkompatiblen Antikörper, die an den GBS-Toxinrezeptor binden, oder
(ii) T-Zellen aus einem Säugetier, die mit einem GBS-Toxinrezeptor kultiviert wurden, oder
(iii) eine Menge eines GBS-Toxins, das ausreicht, um eine Antwort zu induzieren, wobei die Menge an GBS Toxin für eine einzelne Dosis von wenigstens ungefähr 5 µg/kg Körpergewicht vorgesehen ist.

2. Eine Zusammensetzung, enthaltend wenigstens ein immunogenes Fragment eines oder mehrerer Toxinrezeptoren aus β-hämolytischen Streptokokken der Gruppe B (GBS), wobei wenigstens ein immunogenes Fragment im Wesentlichen mit den folgenden Resten der SEQ ID Nr. 2 identisch ist: 49 bis 63 (Hab1), 112 bis 125 (Hab2) oder 8 bis 28 (Hab3).

3. Die Zusammensetzung gemäß Anspruch 1 oder 2, wobei der eine oder die mehreren GBS-Toxinrezeptoren oder immunogenen Fragmente davon in einer Menge vorliegen, die zum Schutz gegen oder zum Mildern eines pathologischen angiogenen Zustandes wirksam ist.

4. Die Zusammensetzung gemäß einem der Ansprüche 1 bis 3, wobei wenigstens eines der Fragmente isoliert vorliegt.

5. Die Zusammensetzung gemäß einem der Ansprüche 1 bis 4, außerdem enthaltend einen pharmazeutisch annehmbaren Trägerstoff.

6. Die Zusammensetzung gemäß einem der Ansprüche 1 bis 5, wobei das Adjuvans ausgewählt ist aus der Gruppe, bestehend aus einer Wasser-in-Öl-Zusammensetzung, Freund'schem Adjuvans, QS21, IL-12 und Interferon-γ.

7. Die Zusammensetzung gemäß einem der Ansprüche 4 bis 6, wobei einer der isolierten GBS-Toxinrezeptoren oder eines der Fragmente davon mit einem Proteinträger konjugiert oder verknüpft ist.

8. Die Zusammensetzung gemäß Anspruch 7, wobei der Proteinträger ein Molekül ist, ausgewählt aus der Gruppe, bestehend aus dem Keyhole-Limpet-Hämocyanin (KLH), Rinderserumalbumin (BSA), Ovalbumin, humanem Serumalbumin, humanem γ-Globulin, Huhn-Immunglobulin G, Rinder-γ-Globulin und Tetanustoxoid.

9. Die Zusammensetzung gemäß einem der Ansprüche 1 bis 8, wobei wenigstens einer der GBS-Toxinrezeptoren oder Fragmente davon glykosyliert ist.

10. Die Zusammensetzung gemäß einem der Ansprüche 1 bis 9, wobei wenigstens einer der isolierten GBS-Toxinrezeptoren oder der Fragmente davon rekombinant oder synthetisch ist.

11. Die Zusammensetzung gemäß einem der Ansprüche 1 bis 10, wobei wenigstens ein GBS-Toxinrezeptor im Wesentlichen identisch ist mit SEQ ID Nr. 2.

12. Die Zusammensetzung gemäß Anspruch 11, wobei wenigstens ein GBS-Toxinrezeptor mit SEQ ID Nr. 2 identisch ist oder SEQ ID Nr. 2 mit wenigstens einer konservativen Aminosäuresubstitution entspricht.

13. Die Zusammensetzung gemäß einem der Ansprüche 1 bis 12, außerdem enthaltend wenigstens einen GBS-Toxinrezeptor, der im Wesentlichen zu SEQ ID Nr. 4 identisch ist.

14. Die Zusammensetzung gemäß Anspruch 13, wobei der GBS-Toxinrezeptor mit SEQ ID Nr. 4 identisch ist oder SEQ ID Nr. 4 mit wenigstens einer konservativen Aminosäuresubstitution oder einem Fragment davon entspricht.

15. Die Zusammensetzung gemäß einem der Ansprüche 1 bis 14, wobei wenigstens ein immunogenes Fragment mit einem Teil der SEQ ID Nr. 4 im Wesentlichen identisch ist.

16. Die Zusammensetzung gemäß Anspruch 15, wobei wenigstens ein immunogenes Fragment mit den folgenden Resten der SEQ ID Nr. 4 im Wesentlichen identisch ist: 8 bis 22 (p55a), 9 bis 35 (p56a) oder 71 bis 84 (p57a).

17. Die Zusammensetzung gemäß einem der Ansprüche 1 bis 16, wobei jeder Antikörper ein monoklonaler Antikörper ist.

18. Die Zusammensetzung gemäß einem der Ansprüche 1 bis 17, wobei jeder Antikörper aus einem polyklonalen Serum erhalten wurde.

19. Die Zusammensetzung gemäß einem der Ansprüche 1 bis 18, wobei wenigstens einer der Antikörper außerdem ein cytotoxisches Mittel umfasst.

20. Die Zusammensetzung gemäß einem der Ansprüche 1 bis 19 zur Verwendung als Impfstoff oder als ein Medikament.

21. Die Zusammensetzung gemäß Anspruch 20 zur Vorbeugung, Milderung oder zur Behandlung eines pathologischen angiogenen Zustandes, der ausgewählt ist aus der Gruppe, bestehend aus Krebs, Narbenbildung während der Wundheilung, einer Gliose während der Wiederherstellung einer Nervenverletzung, chronischen Wunden, Keloiden, Reperfusionsschaden, rheumatoider Arthritis, Arteriosklerose, Osteoarthritis und Psoriasis.

22. Die Zusammensetzung gemäß Anspruch 20 oder 21 zur Behandlung eines Säugetieres.

23. Verwendung der Zusammensetzung gemäß einem der Ansprüche 1 bis 22 für die Herstellung eines Impfstoffes oder eines Medikaments für die Behandlung eines Säugetiers, wobei das normale Gewebe des Säugetiers den GBS-Toxinrezeptor nicht enthält.

24. Die Zusammensetzung gemäß Anspruch 22 oder die Verwendung gemäß Anspruch 23, wobei das Säugetier den pathologischen angiogenen Zustand zum Zeitpunkt des Verabreichungsschrittes nicht aufweist.

25. Die Zusammensetzung gemäß Anspruch 22 oder die Verwendung gemäß Anspruch 23, wobei das Säugetier zum Zeitpunkt des Verabreichungsschrittes den pathologischen angiogenen Zustand aufweist.

26. Die Zusammensetzung gemäß einem der Ansprüche 1 bis 25, wobei die Menge an GBS-Toxin für eine einzelne Dosis von wenigstens ungefähr 15 µg/kg Körpergewicht vorgesehen ist.

27. Die Zusammensetzung gemäß Anspruch 26, wobei die Menge an GBS-Toxin für eine einzelne Dosis von wenigstens ungefähr 20 µg/kg Körpergewicht vorgesehen ist.

28. Ein Verfahren zur Herstellung einer Zusammensetzung gemäß einem der Ansprüche 1 bis 27, umfassend: Bereitstellen wenigstens eines GBS-Toxinrezeptors oder eines immunogenen Fragmentes davon, wie in Anspruch 1 definiert, und Formulieren des Rezeptors oder Fragmentes in einem pharmazeutisch akzeptablem Trägerstoff und Bereitstellen eines Adjuvans.

## Revendications

1. Composition comprenant un ou plusieurs récepteurs de toxines de streptocoques β-hémolytiques du groupe B (GBS), ou des fragments immunogènes de ceux-ci, et un adjuvant,
dans laquelle au moins un fragment immunogène présente une forte identité avec les résidus suivants de la séquence représentée par la SEQ ID n° 2 : 49 à 63 (Hab1), 112 à 125 (Hab2) ou 8 à 28 (Hab3), comprenant éventuellement encore
(i) une quantité efficace d'un ou de plusieurs anticorps compatibles au plan immunitaire, qui se lient à un récepteur de toxines de GBS, ou
(ii) des lymphocytes T provenant d'un mammifère, qui ont été mis en culture en présence d'un récepteur de toxines de GBS, ou
(iii) une quantité de toxine de GBS suffisante pour induire une réponse, dans laquelle la quantité de toxine de GBS est prévue pour un dosage unique d'au moins environ 5 µg/kg de poids corporel.

2. Composition comprenant au moins un fragment immunogène d'un ou de plusieurs récepteurs de toxines de streptocoques β-hémolytique du groupe B (GBS), dans laquelle au moins un fragment immunogène présente une forte identité avec les résidus suivants de la séquence représentée par la SEQ ID n° 2 : 49 à 63 (Hab1), 112 à 125 (Hab2) ou 8 à 28 (Hab3).

3. Composition selon la revendication 1 ou 2, dans laquelle les un ou plusieurs récepteurs de toxines de GBS, ou fragments immunogènes de ceux-ci, se présentent en quantité efficace pour offrir une protection contre un état angiogène pathologique ou pour l'atténuer.

4. Composition selon l'une quelconque des revendications 1 à 3, dans laquelle au moins l'un des fragments est isolé.

5. Composition selon l'une quelconque des revendications 1 à 4, comprenant en outre un excipient acceptable au plan pharmaceutique.

6. Composition selon l'une quelconque des revendications 1 à 5, dans laquelle ledit adjuvant est sélectionné dans le groupe constitué d'une composition eau dans l'huile, d'un adjuvant de Freund, de QS21, d'IL-12 et d'interféron gamma.

7. Composition selon l'une quelconque des revendications 4 à 6, dans laquelle l'un des récepteurs de toxines de GBS isolés, ou l'un de ses fragments, est conjugué ou fixé à un support de protéine.

8. Composition selon la revendication 7, dans laquelle le support de protéine est une molécule sélectionnée dans le groupe constitué de l'hémocyanine de patelle (KLH), de l'albumine de sérum bovin (BSA), de l'ovalbumine, de l'albumine de sérum humain, de la gammaglobuline humaine, de l'immunoglobuline G de poulet, de la gammaglobuline bovine et de l'anatoxine tétanique.

9. Composition selon l'une quelconque des revendications 1 à 8, dans laquelle au moins l'un des récepteurs de toxines de GBS, ou l'un de ses fragments, est glycosylé.

10. Composition selon l'une quelconque des revendications 1 à 9, dans laquelle au moins l'un des récepteurs de toxines de GBS isolés, ou l'un de ses fragments, est recombinant ou synthétique.

11. Composition selon l'une quelconque des revendications 1 à 10, dans laquelle au moins un récepteur de toxines de GBS présente une forte identité avec la séquence représentée par la SEQ ID n° 2.

12. Composition selon la revendication 11, dans laquelle au moins un récepteur de toxines de GBS est identique à la séquence représentée par la SEQ ID n° 2, ou est représenté par la SEQ ID n° 2 contenant au moins une substitution d'acides aminés conservatrice.

13. Composition selon l'une quelconque des revendications 1 à 12, comprenant en outre au moins un récepteur de toxines de GBS qui présente une forte identité avec la séquence représentée par la SEQ ID n° 4.

14. Composition selon la revendication 13, dans laquelle le récepteur de toxines de GBS est identique à la séquence représentée par la SEQ ID n° 4, ou est représenté par la SEQ ID n° 4 contenant au moins une substitution d'acides aminés conservatrice, ou représente l'un de leurs fragments.

15. Composition selon l'une quelconque des revendications 1 à 14, dans laquelle au moins un fragment immunogène présente une forte identité avec une partie de la séquence représentée par la SEQ ID n° 4.

16. Composition selon la revendication 15, dans laquelle au moins un fragment immunogène présente une forte identité avec les résidus suivants de la séquence représentée par la SEQ ID n° 4 : 8 à 22 (p55a), 9 à 35 (p56a) ou 71 à 84 (p57a).

17. Composition selon l'une quelconque des revendications 1 à 16, dans laquelle chaque anticorps est un anticorps monoclonal.

18. Composition selon l'une quelconque des revendications 1 à 17, dans laquelle chaque anticorps est obtenu à partir d'un sérum polyclonal.

19. Composition selon l'une quelconque des revendications 1 à 18, dans laquelle au moins l'un des anticorps comprend, en outre, un agent cytotoxique.

20. Composition selon l'une quelconque des revendications 1 à 19, pour un usage comme vaccin ou comme médicament.

21. Composition selon la revendication 20, destinée à prévenir, atténuer ou traiter un état angiogène pathologique qui est sélectionné dans le groupe constitué du cancer, de la cicatrisation d'une plaie, de la gliose se produisant au cours de la réparation d'une lésion nerveuse, des blessures chroniques, des chéloïdes, d'une lésion de reperfusion, de l'arthrite rhumatoïde, de l'athérosclérose, de l'ostéoarthrite et du psoriasis.

22. Composition selon la revendication 20 ou 21, destinée à traiter un mammifère.

23. Utilisation de la composition selon l'une quelconque des revendications 1 à 22, pour la préparation d'un vaccin ou d'un médicament destiné à traiter un mammifère, dans laquelle le tissu normal du mammifère ne contient pas le récepteur de toxines de GBS.

24. Composition selon la revendication 22 ou utilisation selon la revendication 23, dans laquelle le mammifère n'est pas atteint de l'état angiogène pathologique au moment de l'étape d'administration.

25. Composition selon la revendication 22 ou utilisation selon la revendication 23, dans laquelle le mammifère est atteint de l'état angiogène pathologique au moment de l'étape d'administration.

26. Composition selon l'une quelconque des revendications 1 à 25, dans laquelle la quantité de toxine de GBS est prévue pour un dosage unique d'au moins environ 15 µg/kg de poids corporel.

27. Composition selon la revendication 26, dans laquelle la quantité de toxines de GBS est prévue pour un dosage unique d'au moins environ 20 µg/kg de poids corporel.

28. Procédé de production d'une composition selon
l'une quelconque des revendications 1 à 27, comprenant les étapes consistant à :
mettre en oeuvre au moins un récepteur de toxines de GBS ou un fragment immunogène de celui-ci selon la revendication 1 et formuler le récepteur ou le fragment dans un excipient acceptable au plan pharmaceutique, et fournir un adjuvant.
